(19) Europäisches Patentamt
European Patent Office
Office européen des brevets

(11) EP 4 578 857 A1

(12) EUROPEAN PATENT APPLICATION
published in accordance with Art. 153(4) EPC

(43) Date of publication:
02.07.2025 Bulletin 2025/27

(21) Application number: 24786343.4

(22) Date of filing: 10.07.2024

(51) International Patent Classification (IPC):
C07D 471/04 (2006.01) A01N 43/90 (2006.01)
A01P 1/00 (2006.01) A01P 3/00 (2006.01)

(86) International application number:
PCT/CN2024/104657

(87) International publication number:
WO 2025/102783 (22.05.2025 Gazette 2025/21)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 16.11.2023 CN 202311530160
16.11.2023 CN 202311530564

(71) Applicant: Nankai University
Tianjin 300071 (CN)

(72) Inventors:
- WANG, Qingmin
Tianjin 300071 (CN)
- SONG, Hongjian
Tianjin 300071 (CN)
- ZHOU, Pan
Tianjin 300071 (CN)
- ZHANG, Jingjing
Tianjin 300071 (CN)
- LIU, Yuxiu
Tianjin 300071 (CN)

(74) Representative: Ström & Gulliksson AB
P.O. Box 4188
203 13 Malmö (SE)

# (54) CRYPTOLEPINE SALT DERIVATIVE, AND PREPARATION METHOD THEREFOR AND USE THEREOF

(57) The present invention belongs to the technical field of agriculture protection and, in particular, relates to a cryptolepine salt derivative, a preparation method, and use thereof. The cryptolepine salt derivative is shown in formula (I):

formula (I) 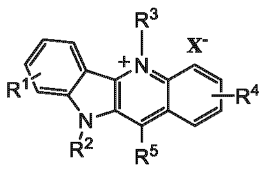 .

By introducing different substituents and using substituents to regulate the electricity and solubility of the cryptolepine salt derivative, the bioactivity of the cryptolepine salt derivative is improved, so that the obtained cryptolepine salt derivative has good plant virus prevention and control effect and bactericidal activity.

EP 4 578 857 A1

## Description

### Cross Reference to Related Applications

This application claims priority to Chinese Application No.202311530160.2 and No.202311530564.1, filed on November 16, 2023, which is specifically and entirely incorporated by reference.

### Technical Field

The present invention belongs to the technical field of agriculture protection and, in particular, relates to a cryptolepine salt derivative, a preparation method and use thereof.

### Background

In 1951, Gellert first isolated the Cryptolepine with indoloquinoline structure from Cryptolepissanguinolenta. The current study found that cryptolepine has comparable activity against Plasmodium falciparum to Chloroquine. And it has been proved that the derivative of cryptolepine have certain drug effects, specifically: 1. There have been studies to synthesize 11-position aniline indole quinoline derivative and show potential anti-tumor activity through in vivo and in vitro experiments. 2. It has been found that cryptolepine salt derivative containing aniline structure have a significant inhibitory effect on methicillin-resistant Staphylococcus aureus. Although there have been many methods for the synthesis of cryptolepine salt derivatives, there are generally shortcomings such as long synthetic route, low yield and harsh reaction conditions. And, so far, there is no use of cryptolepine salt derivatives in the prevention and control of plant viruses and sterilization.

### Disclosure of Invention

The purpose of the present invention is to overcome the shortcomings of the prior art and provide a cryptolepine salt derivative, preparation method and use thereof, wherein the cryptolepine salt derivative has good plant virus prevention and control effect and bactericidal activity.

In order to achieve the above-mentioned object, A second objective of the present invention is to provide a preparation method of the cryptolepine salt derivative.

In the first aspect, the present invention provides a cryptolepine salt derivative with a chemical structure shown in formula (I),

formula (I) 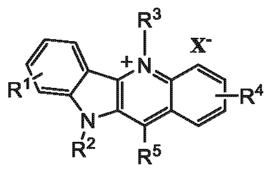 ;

wherein

$R^1$ is selected from at least one group consisting of hydrogen, C1-C6 alkyl, C1-C6 alkoxy, halogen, and cyano;
$R^2$ is selected from at least one group consisting of hydrogen and protecting group;
$R^3$ is selected from at least one group consisting of substituted or unsubstituted C1-C6 alkyl, and C6-C20 aromatic group; wherein substituent of substituted C1-C6 alkyl is selected from at least one group consisting of C3-C6 cycloalkyl;
$R^1$ is selected from at least one group consisting of hydrogen, C1-C6 alkyl, C1-C6 alkoxy, halogen, C1-C6 fluoroalkyl, and C2-C5 ester group;
$R^5$ is selected from at least one group consisting of hydrogen, and -NHR; wherein R is selected from at least one group consisting of substituted or unsubstituted C1-C20 alkyl, C3-C6 cycloalkyl, C3-C12 azacyclic alkyl, and C3-C12 azacyclic alkyl substituted with C1-C6 alkyl; wherein substituent of substituted C1-C20 alkyl is selected from at least one group consisting of C3-C6 cycloalkyl, amino, amino substituted with C1-C12 alkyl, C3-C12 cycloamine group, C3-C12 azacyclic alkyl, and C3-C12 azacyclic alkyl substituted with C1-C6 alkyl;
$X^-$ is selected from salt-forming anion.

In the second aspect, the present invention provides a preparation method of the above-mentioned cryptolepine

salt derivative, comprising:

method (1): when $R^5$ is hydrogen, in presence of catalyst and formylation reagent, making compound A undergo electrophilic substitution reaction, thus to obtain the cryptolepine salt derivative;

method (2): when $R^5$ is selected from -NHR, making compound B undergo nucleophilic substitution reaction with $RNH_2$ to obtain the cryptolepine salt derivative.

compound A 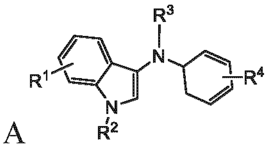 , compound B 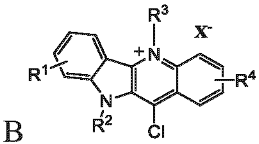 .

In the third aspect, the present invention provides use of the above-mentioned cryptolepine salt derivative in prevention and control of plant viruse.

In the fourth aspect, the present invention provides use of the above-mentioned cryptolepine salt derivative in sterilization.

In the present invention, by introducing different substituents and using substituents to regulate the electricity and solubility of the cryptolepine salt derivative, the bioactivity of the cryptolepine salt derivative is improved, so that the obtained cryptolepine salt derivative has good plant virus prevention and control effect and bactericidal activity.

## Detailed Description

The specific embodiments of the present invention are described in further detail below in conjunction with embodiments. To make the purpose, technical solution, and advantages of the present invention clearer, the technical solution in the embodiment of the present invention will be clearly and completely described below. The described embodiments are some embodiments of the present invention, not all embodiments. Based on the embodiments in the present invention, other embodiments obtained by a person skilled in the art without making creative labor belong to the scope of protection of the present invention. Unless otherwise expressly stated, throughout the description and claims, the term "including" or its transformations such as "contains" or "includes", etc., will be understood to include the stated components and steps, without excluding the existence of other substance components or steps.

In the present invention, the dotted line in a structure indicates the bonding site and represents the bond, such as

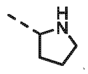

In the present invention, "substituted or unsubstituted C1-C6 alkyl" means that the alkyl has 1 to 6 carbon atoms, and the H on these carbon atoms may or may not be substituted by substituents; When substituted by substituents, the substituted H may be one or more, and when multiple H are substituted, they may be H on the same carbon atom or H on different carbon atoms. The same explanation is be appropriate for all other similar terms.

In the present invention, "C3-C12 azacyclic alkyl substituted with C1-C6 alkyl" means that the azaecyclic has 3 to 12 carbon atoms, and the H on one or more carbon atoms is substituted by alkyl with 1 to 6 carbon atoms, and when more than one H is substituted, the substituted H may be H on the same carbon atom or H on different carbon atoms. The same explanation is be appropriate for all other similar terms.

In the present invention, the alkyls in the expressions "C1-C6 alkyl" and "C1-C20 alkyl" can be either linear chain alkyl or branched chain alkyl. The same explanation is be appropriate for all other similar terms, such as the alkyl part of "C1-C6 alkoxy", which can be linear chain or branched chain.

In the present invention, $R^1$ and $R^4$ of structure formula

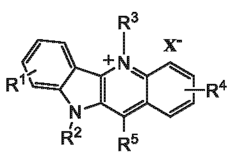 , 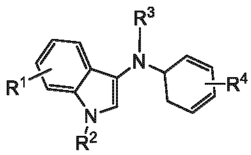 ,

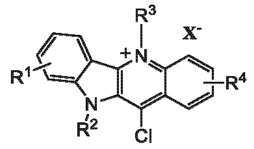

and the same, have multiple substitution sites on the benzene ring and can substitute only one site or multiple sites at the same time, for exmaple

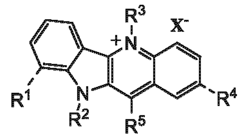

,

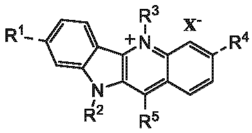

,

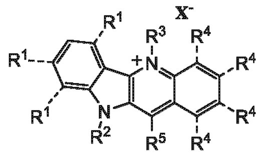

,

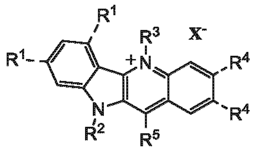

,

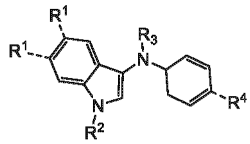

and the same, when multiple sites are substituted, each $R^1$ or $R^4$ is independently selected group and can be the same or different.

The endpoints of ranges and any values disclosed herein are not limited to that exact range or value and should be understood to include values close to those ranges or values. For a numeric range, one or more new numeric ranges may be combined between the end values of the ranges, between the end values of the ranges and individual point values, and between individual point values, which shall be deemed to be specifically disclosed herein.

In order to better explain the technical solution, many specific details are given in the following. Those skilled in the art should understand that this technical solution can be implemented without certain specific details. In some embodiments, raw materials, methods, means, etc. well known to those skilled in the art are not described in detail in order to highlight the subject matter of the invention.

In the first aspect, the present invention provides a cryptolepine salt derivative with a chemical structure shown in formula (I),

formula (I) 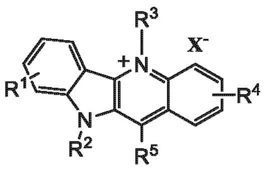 ;

wherein

$R^1$ is selected from at least one group consisting of hydrogen, C1-C6 alkyl, C1-C6 alkoxy, halogen, and cyano;

$R^2$ is selected from at least one group consisting of hydrogen and protecting group;

$R^3$ is selected from at least one group consisting of substituted or unsubstituted C1-C6 alkyl, and C6-C20 aromatic group; wherein substituent of substituted C1-C6 alkyl is selected from at least one group consisting of C3-C6 cycloalkyl;

$R^1$ is selected from at least one group consisting of hydrogen, C1-C6 alkyl, C1-C6 alkoxy, halogen, C1-C6 fluoroalkyl, and C2-C5 ester group;

$R^5$ is selected from at least one group consisting of hydrogen, and -NHR; wherein R is selected from at least one group consisting of substituted or unsubstituted C1-C20 alkyl, C3-C6 cycloalkyl, C3-C12 azacyclic alkyl, and C3-C12 azacyclic alkyl substituted with C1-C6 alkyl; wherein substituent of substituted C1-C20 alkyl is selected from at least one group consisting of C3-C6 cycloalkyl, amino, amino substituted with C1-C12 alkyl, C3-C12 cycloamine group, C3-C12 azacyclic alkyl, and C3-C12 azacyclic alkyl substituted with C1-C6 alkyl;

$X^-$ is selected from salt-forming anion.

In the present invention, when $R^2$ is selected from the protecting group, the protecting group refers to the

protecting group of amine active hydrogen suitable for the structure shown in formula (I).

In the present invention, in order to obtain a cryptolepine salt derivative having better plant virus prevention and control effect and bactericidal effect, it is necessary to further select the substituents.

According to a preferred embodiment of the present invention,

$R^1$ is selected from at least one group consisting of hydrogen, C1-C3 alkyl, C1-C3 alkoxy, halogen, and cyano;
$R^2$ is selected from at least one group consisting of hydrogen, p-toluenesulfonyl, benzenesulfonyl, benzyloxycarbonyl, tert-butoxycarbonyl and benzyl;
$R^3$ is selected from at least one group consisting of substituted or unsubstituted C1-C6 alkyl, and C6-C12 aromatic group; wherein substituent of substituted C1-C6 alkyl is selected from at least one group consisting of C3-C6 cycloalkyl;
$R^1$ is selected from at least one group consisting of hydrogen, C1-C3 alkyl, C1-C3 alkoxy, halogen, C1-C3 perfluoroalkyl, and C2-C5 ester group;
$R^5$ is selected from at least one group consisting of hydrogen and -NHR; wherein R is selected from at least one group consisting of substituted or unsubstituted C1-C12 alkyl, C3-C6 cycloalkyl, C3-C6 azacyclic alkyl, and C3-C6 azacyclic alkyl substituted with C1-C3 alkyl; wherein substituent of substituted C1-C12 alkyl is selected from at least one group consisting of C3-C6 cycloalkyl, amino, amino substituted with C1-C6 alkyl, C3-C6 cycloamine group, C3-C6 azacyclic alkyl, and C3-C6 azacyclic alkyl substituted with C1-C3 alkyl;
$X^-$ is selected from at least one group consisting of $Cl^-$, $Br^-$, $I^-$, $CH_3COO^-$, $NO_3^-$, $HSO_4^-$, $H_2PO_4^-$, $BF_4^-$, and $SbF_6^-$.

In the present invention, the C2-C5 ester group can be expressed as $-COOR^0$, where $R^0$ is selected from methyl, ethyl, n-propyl, isopropyl, n-butyl, isobutyl and tert-butyl.

According to a preferred embodiment of the present invention, wherein,

Situation 1: when $X^-$ is $Cl^-$, it is satisfied as following structure:

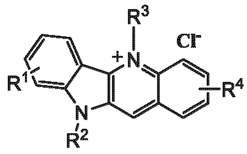

;

$R^1$ is selected from at least one group consisting of hydrogen, methyl, methoxy, F, Cl, Br, I, and cyano; $R^2$ is selected from at least one group consisting of hydrogen, p-toluenesulfonyl, and benzenesulfonyl; $R^3$ is selected from at least one group consisting of methyl, 2,2-dimethylpropyl, cyclopropylmethyl, cyclohexylmethyl, and benzyl; $R^4$ is selected from at least one group consisting of hydrogen, methyl, methoxy, F, Cl, Br, I, trifluoromethyl, and methyl formate group; $R^5$ is hydrogen.

Situation 2: when $X^-$ is $I^-$, it is satisfied as following structure:

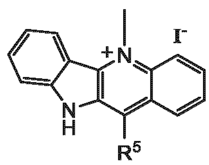

;

$R^1$, $R^2$, $R^1$ is hydrogen, $R^3$ is methyl, $R^5$ is selected from at least one of -NHR, wherein R is selected from at least one group consisting of substituted or unsubstituted C1-C12 alkyl, C3-C6 cycloalkyl, C3-C6 azacyclic alkyl, and C3-C6 azacyclic alkyl substituted with C1-C3 alkyl; wherein substituent of substituted C1-C12 alkyl is selected from at least one group consisting of C3-C6 cycloalkyl, amino, amino substituted with C1-C6 alkyl, C3-C6 cycloamine group, C3-C6 azacyclic alkyl, and C3-C6 azacyclic alkyl substituted with C1-C3 alkyl.

Preferably, referring to the situation 2, when $X^-$ is $I^-$, it is satisfied that: $R^1$, $R^2$, $R^4$ is hydrogen, $R^3$ is methyl, $R^5$ is selected from -NHR, wherein R comprises at least one group consisting of linear or branched C3-C12 alkyl, C3-C6 cycloalkyl, methyl substituted with C3-C6 cycloalkyl, propyl substituted with C3-C6 cycloalkyl, substituted or unsubstituted C2-C6 alkylamino, tetrahydropyrrole group, piperidyl, piperidyl methyl, n-methylpiperidyl, and n-methyl piperidine methyl; wherein substituent of substituted C2-C6 alkylamino is selected from at least one group consisting of methyl, ethyl, and

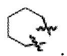

.

For the choice of R, further preferably, R is selected from at least one group consisting of n-propyl, n-amyl, isoamyl, n-dodecyl, 2-ethylhexyl, cyclopropyl, cyclopentyl, cyclohexyl, cyclopropylmethyl, cycloamylmethyl, cyclohexylmethyl, cyclopropylethyl, cycloamylethyl, cyclohexylethyl, cyclopropylpropyl, cycloamylpropyl, cyclohexyl propyl, aminomethyl, aminoethyl, aminopropyl, aminobutyl, 2-aminopropyl, 2-methyl-2-amino-propyl, $-CH_2CH_2-NH-CH_2CH_3$, $-CH_2CH_2CH_2-NH-CH_3$, $-CH_2C(CH_3)_2-CH_2-N(CH_3)_2$,

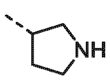, 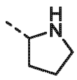, 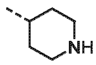, 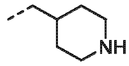,

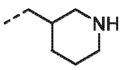, 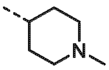, 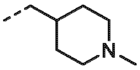, 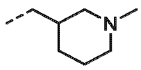, 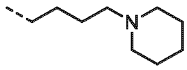, and 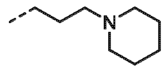.

According to another preferred embodiment of the present invention, wherein, when $X^-$ is $Cl^-$, it is satisfied that: $R^1$ is selected from at least one group consisting of hydrogen, F, Cl, Br, and I; $R^2$ is selected from at least one group consisting of hydrogen, p-toluenesulfonyl, and benzenesulfonyl; $R^3$ is selected from at least one group consisting of methyl, 2,2-dimethylpropyl, cyclopropylmethyl, cyclohexylmethyl, and benzyl; $R^4$ is selected from at least one group consisting of hydrogen, methyl, Br, and trifluoromethyl; $R^5$ is hydrogen.

According to another preferred embodiment of the present invention, wherein, when $X^-$ is $I^-$, it is satisfied that: $R^1$ is hydrogen, $R^2$ is hydrogen, $R^3$ is methyl, $R^4$ is hydrogen, $R^5$ is selected from at least one of hydrogen and -NHR, wherein R is selected from at least one group consisting of 2-ethylhexyl, cyclopropylmethyl, aminopropyl, aminobutyl, isoamyl, 2-aminopropyl,

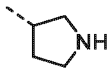,

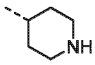, 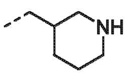, 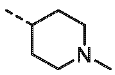, and 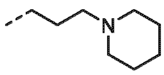.

According to a further preferred embodiment of the present invention, the cryptolepine salt derivative comprises at least one of the compounds shown in formula (I-1) to formula (I-71):

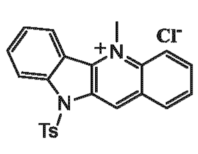
I-1

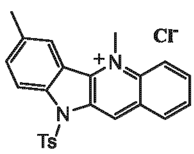
I-2

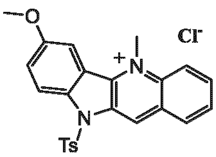
I-3

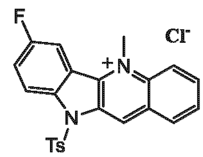
I-4

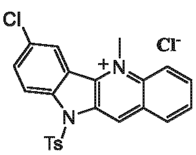
I-5

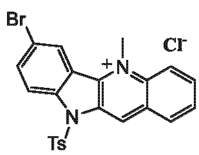
I-6

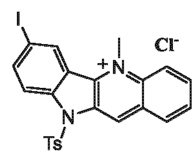
I-7

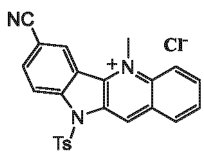
I-8

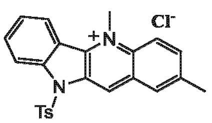
I-9

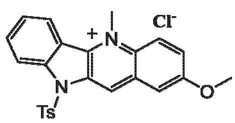
I-10

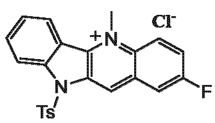
I-11

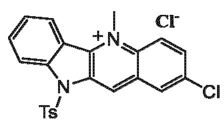
I-12

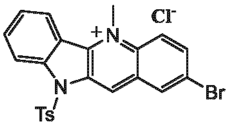
I-13

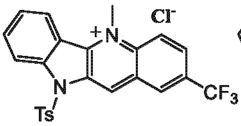
I-14

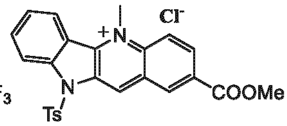
I-15

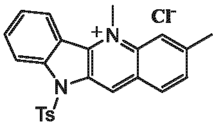
I-16

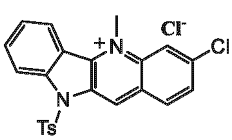
I-17

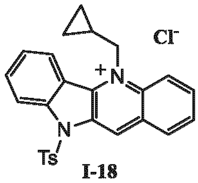
I-18

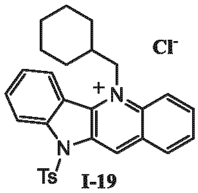
I-19

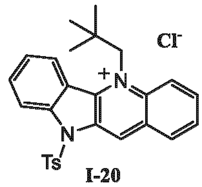
I-20

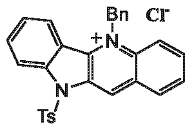
I-21

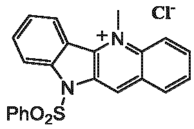
I-22

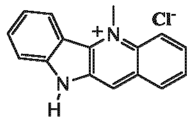
I-23

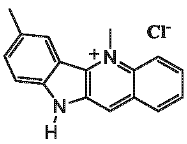
I-24

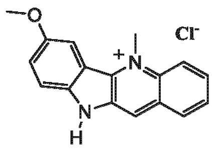
I-25

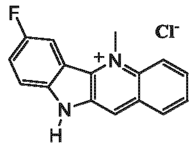
I-26

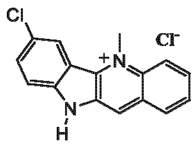
I-27

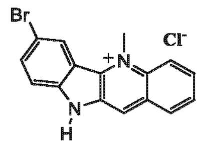
I-28

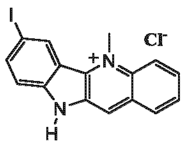
I-29

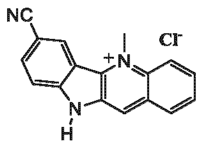
I-30

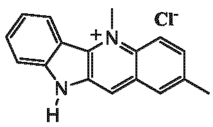
I-31

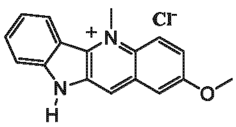
I-32

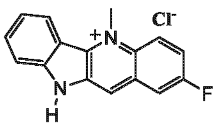
I-33

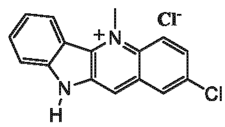
I-34

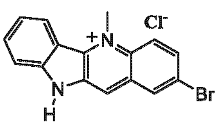
I-35

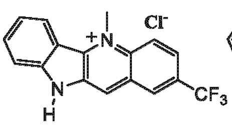
I-36

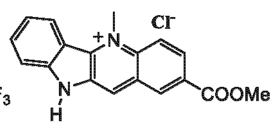
I-37

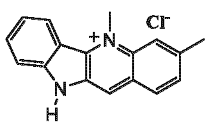
I-38

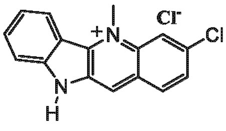
I-39

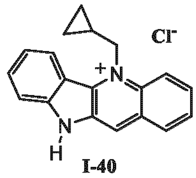
I-40

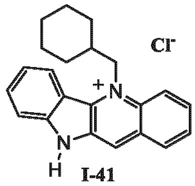
I-41

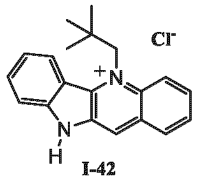
I-42

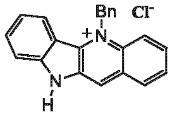
I-43

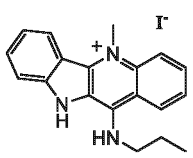
I-44

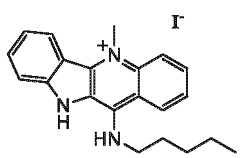
I-45

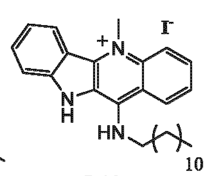
I-46

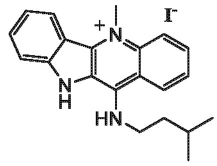
I-47

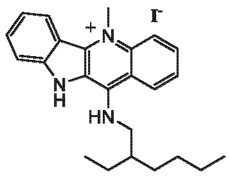
I-48

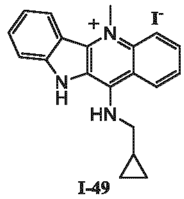
I-49

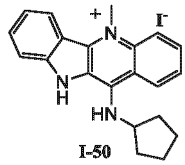
I-50

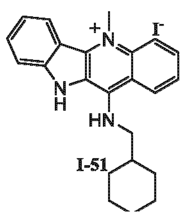
I-51

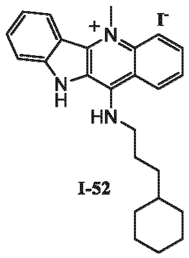
I-52

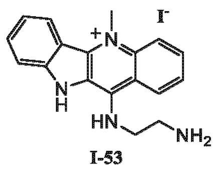
I-53

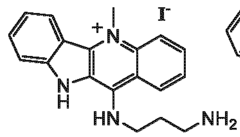
I-54

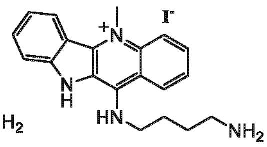
I-55

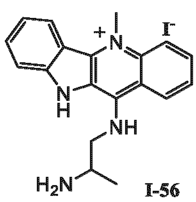
I-56

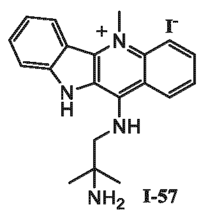
I-57

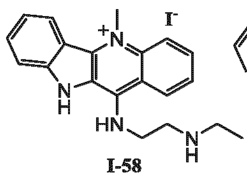
I-58

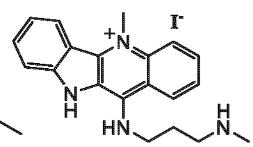
I-59

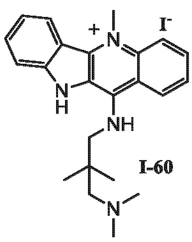
I-60

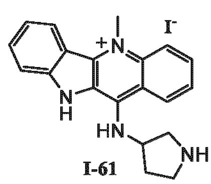
I-61

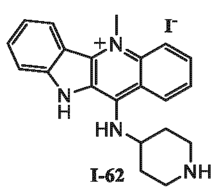
I-62

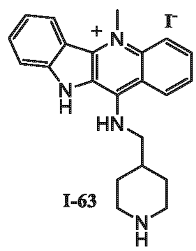
I-63

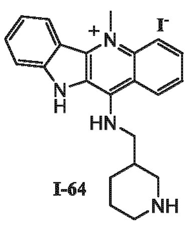
I-64

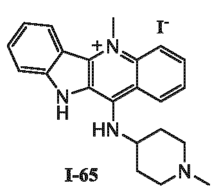
I-65

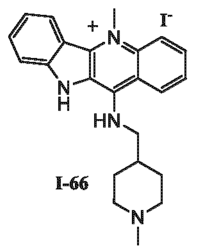
I-66

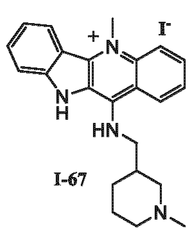
I-67

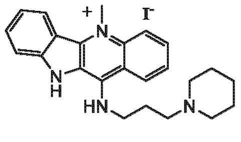
I-68
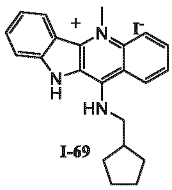
I-69
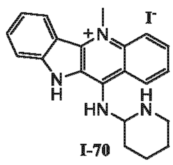
I-70
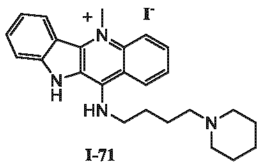
I-71

According to a particularly preferred embodiment of the present invention, wherein, the cryptolepine salt derivative comprises at least one of the following compounds:

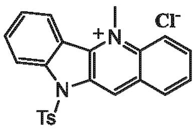
I-1
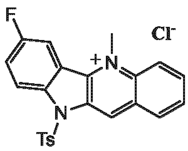
I-4
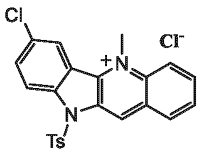
I-5
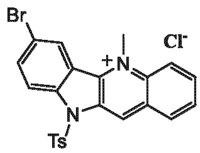
I-6
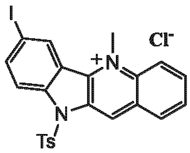
I-7
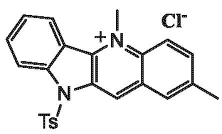
I-9

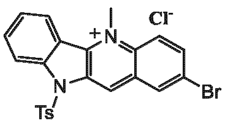
I-13
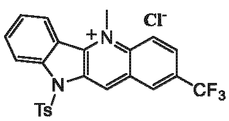
I-14
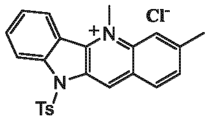
I-16
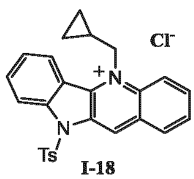
I-18
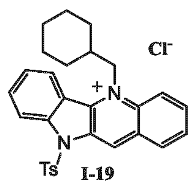
I-19
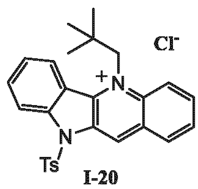
I-20

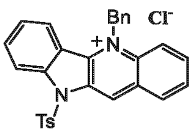
I-21
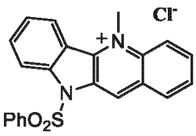
I-22
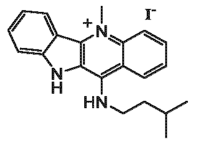
I-47
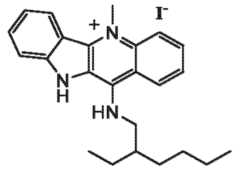
I-48
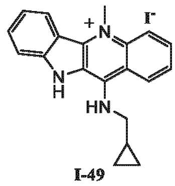
I-49
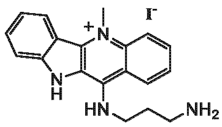
I-54

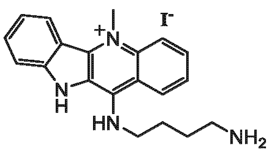
I-55
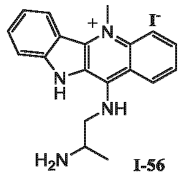
I-56
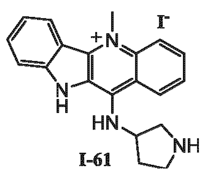
I-61
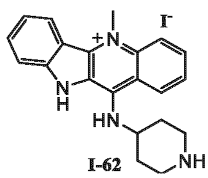
I-62
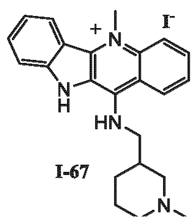
I-67

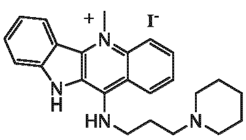
I-68

In the present invention, by introducing different substituents, the substituents can improve the virus prevention and control ability and bactericidal activity of the cryptolepine salt derivative. The cryptolepine salt derivative containing amino structure (that is when $R^5$ is selected from -NHR) introduce primary amines with different substituents at the C-11 site, wherein the primary amines can be used as both hydrogen bond donors and hydrogen bond acceptors to increase the hydrogen bond site between molecule and target protein, and thus to improve the biological activity of the compound.

In the second aspect, the present invention provides a preparation method of the above-mentioned cryptolepine salt derivative, comprising:

method (1): when $R^5$ is hydrogen, in presence of catalyst and formylation reagent, making compound A undergo electrophilic substitution reaction, thus to obtain the cryptolepine salt derivative;

method (2): when $R^5$ is selected from -NHR, making compound B undergo nucleophilic substitution reaction with $RNH_2$ to obtain the cryptolepine salt derivative.

compound A 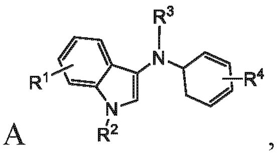 , compound B 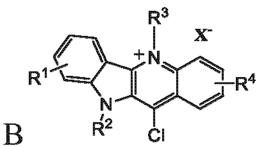 .

According to a preferred embodiment of the present invention, futher, the preparation method of the cryptolepine salt derivative comprising:

method (1) : when $R^5$ is hydrogen, in presence of catalyst and formylation reagent, making compound A undergo electrophilic substitution reaction with imide salt, thus to obtain the cryptolepine salt derivative;

method (2) : when $R^5$ is selected from -NHR, making compound B undergo nucleophilic substitution reaction with $RNH_2$ under a condition of heating reflux by using ethyl acetate as solvent to obtain the cryptolepine salt derivative.

In the present invention, for the method (1), in order to make the reaction better and obtain higher product yield and purity, the reagent involved in the reaction and the reaction conditions need to be controlled.

Preferably, the catalyst is selected from at least one of $POCl_3$, $SOCl_2$, $ZnCl_2$ and $COCl_2$, preferably selected from at least one of $POCl_3$ and $SOCl_2$.

Preferably, the formylation reagent is selected from at least one of N-substituted formamides, preferably selected from at least one of N,N-Dimethylformamide, N-Methyl-N-phenylformamide, N,N-Dipropylformamide, and N-ethyl-N-methylformamide, more preferably selected from at least one of N,N-Dimethylformamide and N-Methyl-N-phenylformamide.

Preferably, molar ratio of the compound A and the catalyst is 1:1.2-3, preferably is 1:1.5-2.5, for example can be 1:1.5, 1:1.8, 1:2.2, 1:2.5, ect. and range between any of the values thereof. Relative to the above molar ratio range, when the molar ratio of compound A and catalyst is greater than 1:3, the yield of the cryptolepine salt derivative obtained is significantly decreased; when the molar ratio of compound A to catalyst was less than 1:1.2, the yield of the cryptolepine salt derivative also decreased significantly.

Preferably, the compound A is provided in solution when added to the reaction system, wherein concentration of the compound A is 0.2-1mmol/mL, preferably 0.4-0.6mmol/mL, for example, be values of 0.4mmol/mL, 0.5mmol/mL, 0.55mmol/mL, 0.6mmol/mL, ect. and the range between any values thereof. The solvent for dissolving compound A is at least one of the formylation reagents described above.

Preferably, temperature of the electrophilic substitution reaction is -20°C to 30°C, preferably is -16°C to 25°C, for example, the values of -16°C, -10°C, -5°C, 0°C, 5°C, 10°C, 20°C, 25° C, ect. and the range between any values thereof. Time of the electrophilic substitution reaction is 0.5-4h, preferably is 1-2h, for example, can be 1h, 1.5h, 1.8h and 2h, and other values and the range between any values thereof.

According to the present invention, the imide salt is an intermediate

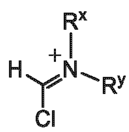

obtained by a reaction of the catalyst and the formylation reagent, wherein $R^x$ and $R^y$ are two substituents on the N position of N-substituted formamide, respectively.

In the present invention, for the method (2), in order to make the reaction better and obtain a higher product yield and purity, the reaction conditions need to be controlled.

Preferably, temperature of the heating reflux is 90-150°C, preferably is 110-130°C, for example, can be 110°C, 115°C, 122°C, 128°C, etc. and the range between any values thereof. Time of the heating reflux is 10-24h, preferably is 15-17h, for example, can be 15h, 16h, 16.5h, 17h and other values and the range between any values thereof.

Preferably, molar ratio of the compound B to $RNH_2$ is 1:1-5, preferably is 1:1-3, for example, the values of 1:1, 1:2, 1:2.5, and 1:3, and other values and the range between any values thereof.

In the third aspect, the present invention provides use of the above-mentioned cryptolepine salt derivative in prevention and control of plant viruse.

In the fourth aspect, the present invention provides use of the above-mentioned cryptolepine salt derivative in sterilization.

The cryptolepine salt derivative provided by the present invention has excellent anti-plant virus activity, and the cryptolepine salt derivative shown in formula (I) exhibits good anti-tobacco Mosaic virus activity. In addition, the cryptolepine salt derivative provided by the invention also has high bactericidal activity, especially against the pathogens causing powdery mildew of cucumber and rice sheath blight disease.

The invention is described in detail in the following embodiments, but the invention is not limited to the following embodiments, the reagent are not specifically stated in the embodiments, and can be obtained by purchase.

In the following examples:
Compounds of examples 1-22 are prepared by using the above method (1), while compounds of examples 23-47 are prepared by using the above method (2).

Compound A is prepared according to the method described in the literature (Tetrahedron Lett. 1998, 39: 6465-6466.).

Compound B is prepared according to the method described in the literature (J. Med. Chem. 1998, 41 (15): 2754-2764; J. Ethnopharmacol. 2005, 100 (1-2): 67-71).

Preparation Examples 1-22

Preparation Examples 1-22 are used to illustrate the preparation method of compound A.

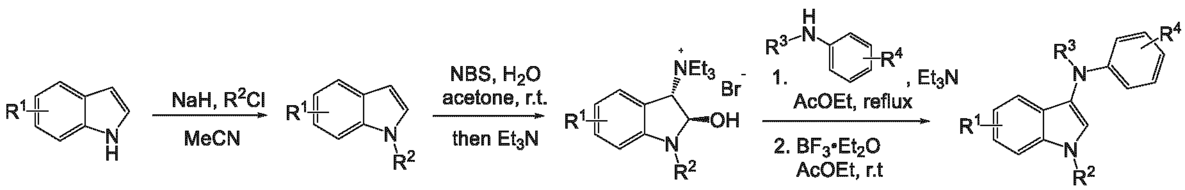


a1 a2 a3 A

30mmol of compound a1 (each compound a1 selected for preparation examples 1-22 is shown in Table 1) was dissolved in 30mL acetonitrile, after slowly adding 40mmol of sodium hydride in batches at 0°C, the reaction system was stirred for 10min and then to return to room temperature, after adding 33mmol $R^2Cl$ (each compound $R^2Cl$ selected for preparation examples 1-22 is shown in Table 1) and until the reaction was completed, the saturated ammonium chloride aqueous solution was added for quenching the reaction, then the reaction system was extracted with ethyl acetate, the obtainded organic phase was combined, washed by saturated salt water, dried with anhydrous sodium sulfate, distilled under reduced pressure, and purified by column chromatography to obtain white solid compound a2.

11mmol of compound a2 and 110mmol of high purity water were dissolved in 110mL of acetone, then 12mmol of NBS (N-Bromosuccinimide) was added until the reaction was completed, then 12mmol of triethylamine was added to the reaction system and stirred for 1h to precipitate a large amount of white solid, then the reaction system was suction filtered, the obtained solid was washed with acetone several times and dried to get compound a3.

5mmol of compound a3, 5.5mmol of

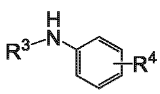

(the specific compound selected for preparation examples 1-22 is shown in Table 1) and 10mmol of triethylamine were dissolved in 100mL of ethyl acetate, then the mixture was heated and refluxed for 6h, and after the reaction was completed, water was added to the reaction system which was latter extracted by ethyl acetate, the obtained organic phase was combined, washed by saturated salt water, dried with anhydrous sodium sulfate. After vacuum distillation, it was dissolving in ethyl acetate, after adding 25mmol of boron trifluoride ether solution, it was reacted at 50°C for 3h and cooled to room temperature after reaction was completed, the reaction system was slowly added with saturated sodium bicarbonate solution, extracted by ethyl acetate, the obtained organic phase was combined, washed by saturated salt water, dried with anhydrous sodium sulfate, distilled under reduced pressure and purified by column chromatography to obtain white solid compound A (see Table 1 for the compound A obtained in preparation examples 1-22 respectively).

Table 1

| serial number | Compound a1 | $R^2Cl$ | 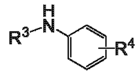 | Compound A |
|---|---|---|---|---|
| Preparation Example 1 | Indole | P-toluenesulfonyl chloride | 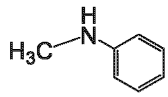 | 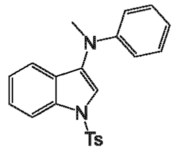 |
| Preparation Example 2 | 5-methylindole | P-toluenesulfonyl chloride | 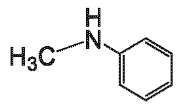 | 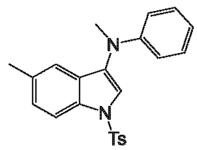 |
| Preparation Example 3 | 5-methoxyindole | P-toluenesulfonyl chloride | 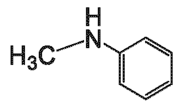 | 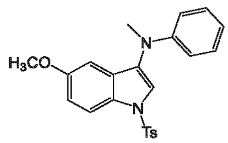 |
| Preparation Example 4 | 5-F-indole | P-toluenesulfonyl chloride | 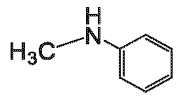 | 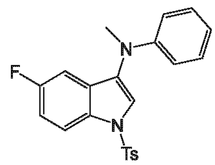 |
| Preparation Example 5 | 5-Cl-indole | P-toluenesulfonyl chloride | 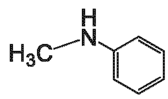 | 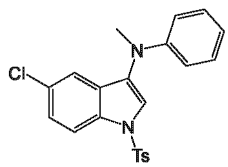 |
| Preparation Example 6 | 5-Br-indole | P-toluenesulfonyl chloride | 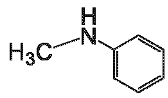 | 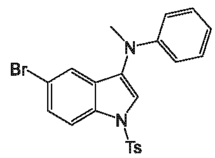 |
| Preparation Example 7 | 5-I-indole | P-toluenesulfonyl chloride | 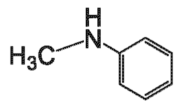 | 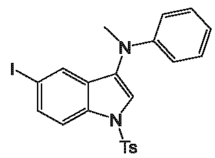 |
| Preparation Example 8 | 5-cyanoindole | P-toluenesulfonyl chloride | 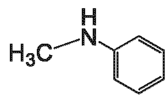 | 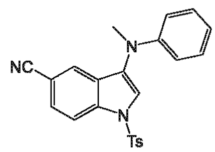 |
| Preparation Example 9 | Indole | P-toluenesulfonyl chloride | 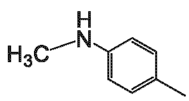 | 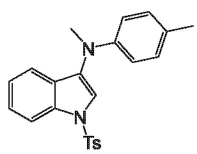 |

(continued)

| serial number | Compound a1 | $R^2Cl$ | 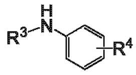 | Compound A |
|---|---|---|---|---|
| Preparation Example 10 | Indole | P-toluene sulfonyl chloride | 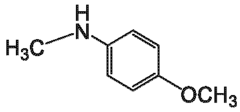 | 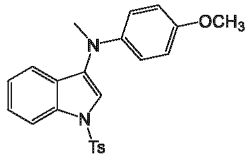 |
| Preparation Example 11 | Indole | P-toluenesulfonyl chloride | 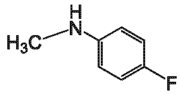 | 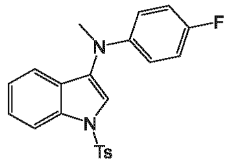 |
| Preparation Example 12 | Indole | P-toluenesulfonyl chloride | 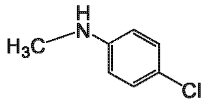 | 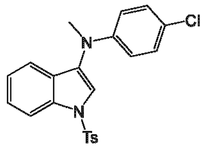 |
| Preparation Example 13 | Indole | P-toluenesulfonyl chloride | 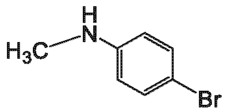 | 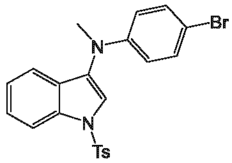 |
| Preparation Example 14 | Indole | P-toluenesulfonyl chloride | 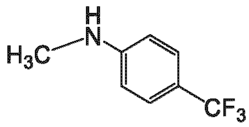 | 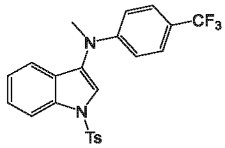 |
| Preparation Example 15 | Indole | P-toluenesulfonyl chloride | 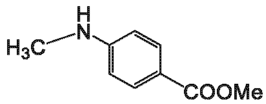 | 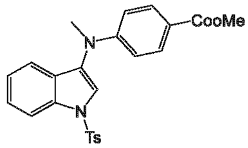 |
| Preparation Example 16 | Indole | P-toluenesulfonyl chloride | 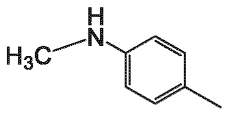 | 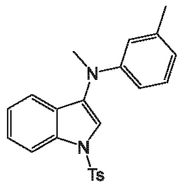 |
| Preparation Example 17 | Indole | P-toluenesulfonyl chloride | 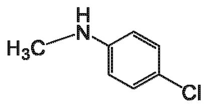 | 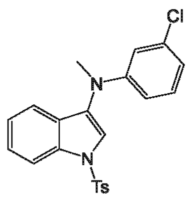 |
| Preparation Example 18 | Indole | P-toluenesulfonyl chloride | 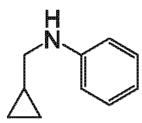 | 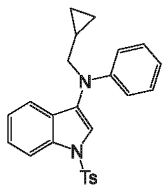 |

(continued)

| serial number | Compound a1 | $R^2Cl$ | 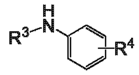 | Compound A |
|---|---|---|---|---|
| Preparation Example 19 | Indole | P-toluenesulfonyl chloride | 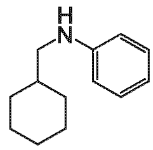 | 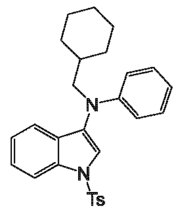 |
| Preparation Example 20 | Indole | P-toluenesulfonyl chloride | 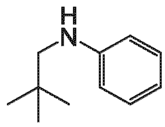 | 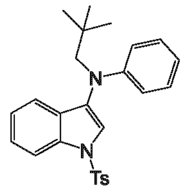 |
| Preparation Example 21 | indole | P-toluenesulfonyl chloride | 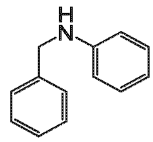 | 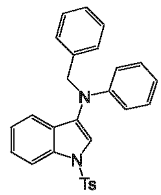 |
| Preparation Example 22 | Indole | Benzene sulfonyl chloride | 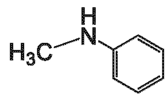 | 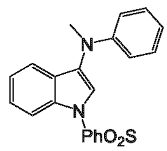 |

Preparation Example 23

Preparation Example 23 is used to illustrate the preparation method of compound B.

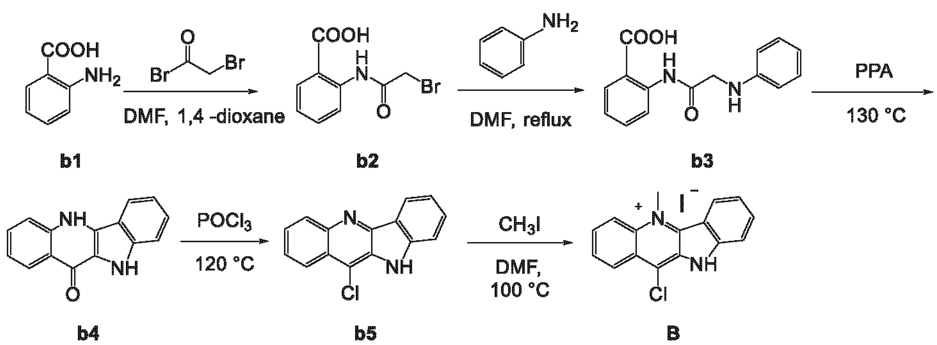


20mmol of compound b1 was dissolved in a mixture solution of 8mL N,N dimethylformamide and 8mL 1, 4-dioxane, after slowly dripped with 24mmol of bromoacetyl bromide at 0°C, the reaction system was stirred overnight at room temperature. After the reaction is completed, the reaction liquid was poured into an appropriate amount of ice water, a large amount of white solid was precipitated, suction filtered, washed five times with a large amount of water, and dried to obtain the compound b2.

19.4mmol of compound b2 was dissolved in 10mL of N,N dimethylformamide, after adding 69.4mmol of aniline, the reaction system was heated and refluxed at 120°C for 18h, then cooled to room temperature after the reaction was completed, by slowly adding 5% potassium hydroxide solution to adjust pH to 11, the reaction system was extracted with methylene chloride several times, the obtained water phase was combined and pH of which was adjusted to 2-3 with 5% hydrobromic acid solution and then standed at room temperature overnight, a large amount of white solid was precipitated,

suction filtered, and dried to obtain compound b3.

150g PPA (polyphosphates) was added to 15.8mmol of compound b3, the mixture was heated at 130°C for 2h, cooled to room temperature after the reaction was completed, the reaction liquid was poured into an appropriate amount of ice water, pH of which was adjusted to neutral with saturated potassium hydroxide solution, extracted by ethyl acetate while the organic phase was combined, washed with saturated salt water, dried with anhydrous sodium sulfate, distilled under reduced pressure and purified by column chromatography to obtain compound b4.

33mL $POCl_3$ (phosphorus oxychloride) was slowly dripped to 13mmol of compound b4, the mixture was heated and reflexed for 2h at 120°C, cooled to room temperature after the reaction was completed, the reaction liquid was slowly poured into appropriate amount of ice water, pH of which was adjusted to neutral with saturated potassium hydroxide solution, extracted by ethyl acetate while the organic phase was combined, and washed with saturated salt water, dried with anhydrous sodium sulfate, distilled under reduced pressure and purified by column chromatography to obtain compound b5.

0.5mmol of compound b5 was dissolved in 2mL N,N dimethylformamide, after adding 1.5mmol of iodomethane, the mixture was heated at 100°C for 8h, then cooled to room temperature after the reaction was completed, though adding ethyl acetate to the mixture, a large amount of solid precipitated, suction filtered, washed with ethyl acetate several times, and dried to obtain compound B.

Examples 1-22

Examples 1-22 are used to illustrate the synthesis of the cryptolepine salt derivatives shown in formulas (I-1) to (I-22).

2mmol $POCl_3$ (phosphorus oxytrichloride) was slowly dripped into 1mL of N,N dimethylformamide at -16°C and stirred for 0.5h. At 0°C, 1mmol of the corresponding compound A (Examples 1-22 respectively adopt compound A obtained from preparation example 1-22 above) dissolved in 2mL N,N dimethylformamide was further added into the above solution and the obtained mixture was reacted at room temperature for 1h. After the reaction was completed, the reaction liquid was poured into ice water, stirred to precipitate the solid, and 10% sodium hydroxide solution was added to adjust the pH to about 10. After standing for 1h, the reaction system was filtered to obtain the cryptolepine salt derivatives as shown in formula (I-1) to Formula (I-22) respectively (the yield, melting point and appearance are detailed in Table 3).

Examples 23-47

Examples 23-47 are used to illustrate the synthesis of the cryptolepine salt derivatives shown in formulas (I-44) to (I-68).

0.5mmol of compound B was weighed and dissolved in 20mL ethyl acetate, 1mmol of corresponding $RNH_2$ (group R of compound $RNH_2$ selected for example 23-47 is shown in Table 2) was added into the above solution and the obtained mixture was heated and refluxed for 16h. After the reaction was completed, it was cooled to room temperature, some solids were precipitated, suction filtered, washed with ethyl acetate several times, and dried to obtain the cryptolepine salt derivatives as shown in formula (I-44) to formula (I-68) respectively (the yield, melting point and appearance are detailed in Table 3).

Table 2

| Example serial number | R |
|---|---|
| Example 23 | n-propyl |
| Example 24 | n-amyl |
| Example 25 | n-dodecyl |
| Example 26 | isoamyl |
| Example 27 | 2-ethylhexyl |
| Example 28 | cyclopropylmethyl |
| Example 29 | cycloamylmethyl |
| Example 30 | cyclohexylmethyl |
| Example 31 | cyclohexylpropyl |
| Example 32 | aminoethyl |
| Example 33 | aminopropyl |

(continued)

| Example serial number | R |
|---|---|
| Example 34 | aminobutyl |
| Example 35 | 2-aminopropyl |
| Example 36 | 2-methyl-2-amino-propyl |
| Example 37 | $-CH_2CH_2-NH-CH_2CH_3$ |
| Example 38 | $-CH_2CH_2CH_2-NH-CH_3$ |
| Example 39 | $-CH_2C(CH_3)_2-CH_2-N(CH_3)_2$ |
| Example 40 | 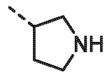 |
| Example 41 | 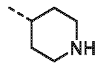 |
| Example 42 | 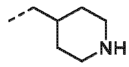 |
| Example 43 | 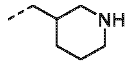 |
| Example 44 | 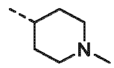 |
| Example 45 | 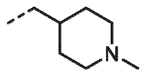 |
| Example 46 | 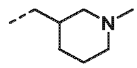 |
| Example 47 | 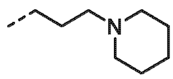 |

Table 3

| Example serial number | Corresponding compound | Product yield /% | Product melting point /°C | Product status |
|---|---|---|---|---|
| Example 1 | Formula (I-1) | 93 | 148-149 | Yellow solid |
| Example 2 | Formula (I-2) | 92 | 151-152 | Yellow solid |
| Example 3 | Formula (I-3) | 99 | 162-163 | Bright orange solid |
| Example 4 | Formula (I-4) | 86 | 160-161 | Bright yellow solid |
| Example 5 | Formula (I-5) | 99 | 158-159 | Bright yellow solid |
| Example 6 | Formula (I-6) | 94 | 154-155 | Yellow solid |
| Example 7 | Formula (I-7) | 92 | 138-139 | Orange-yellow solid |
| Example 8 | Formula (I-8) | 92 | 112-113 | Pale yellow solid |
| Example 9 | Formula (I-9) | 97 | 152-153 | Yellow-green solid |
| Example 10 | Formula (I-10) | 99 | 188-189 | Yellow solid |
| Example 11 | Formula (I-11) | 93 | 153-154 | Yellow solid |
| Example 12 | Formula (I-12) | 87 | 178-179 | Green solid |
| Example 13 | Formula (I-13) | 95 | 167-168 | Yellow solid |

(continued)

| Example serial number | Corresponding compound | Product yield /% | Product melting point /°C | Product status |
|---|---|---|---|---|
| Example 14 | Formula (I-14) | 98 | 165-166 | Bright yellow solid |
| Example 15 | Formula (I-15) | 91 | 157-158 | Yellow-green solid |
| Example 16 | Formula (I-16) | 99 | 141-142 | Yellow-green solid |
| Example 17 | Formula (I-17) | 94 | 154-155 | Yellow solid |
| Example 18 | Formula (I-18) | 99 | 113-114 | Yellow solid |
| Example 19 | Formula (I-19) | 98 | 142-143 | Yellow solid |
| Example 20 | Formula (I-20) | 95 | 127-128 | Yellow solid |
| Example 21 | Formula (I-21) | 99 | 156-157 | Yellow solid |
| Example 22 | Formula (I-22) | 97 | 140-141 | Bright yellow solid |
| Example 23 | Formula (I-44) | 59 | Greater than 250 | Yellow-green solid |
| Example 24 | Formula (I-45) | 60 | 230-231 | Yellow solid |
| Example 25 | Formula (I-46) | 57 | 171-172 | Yellow solid |
| Example 26 | Formula (I-47) | 78 | 238-239 | Yellow solid |
| Example 27 | Formula (I-48) | 72 | 200-201 | Yellow solid |
| Example 28 | Formula (I-49) | 62 | Greater than 250 | Yellow solid |
| Example 29 | Formula (I-50) | 60 | 248-249 | Brown-yellow solid |
| Example 30 | Formula (I-51) | 79 | 243-244 | Yellow solid |
| Example 31 | Formula (I-52) | 51 | 234-235 | Yellow solid |
| Example 32 | Formula (I-53) | 87 | 235-236 | Yellow solid |
| Example 33 | Formula (I-54) | 86 | 198-199 | Yellow solid |
| Example 34 | Formula (I-55) | 84 | 203-204 | Yellow solid |
| Example 35 | Formula (I-56) | 87 | 221-222 | Yellow solid |
| Example 36 | Formula (I-57) | 89 | 252-253 | Yellow solid |
| Example 37 | Formula (I-58) | 49 | 233-234 | Yellow solid |
| Example 38 | Formula (I-59) | 74 | 187-188 | Tangerine solid |
| Example 39 | Formula (I-60) | 64 | 242-243 | Yellow solid |
| Example 40 | Formula (I-61) | 79 | 184-185 | Brown-yellow solid |
| Example 41 | Formula (I-62) | 81 | 245-246 | Orange solid |
| Example 42 | Formula (I-63) | 78 | 217-218 | Yellow solid |
| Example 43 | Formula (I-64) | 82 | 200-201 | Yellow solid |
| Example 44 | Formula (I-65) | 85 | 240-241 | Yellow-green solid |
| Example 45 | Formula (I-66) | 75 | 246-247 | Yellow solid |
| Example 46 | Formula (I-67) | 71 | 177-178 | Yellow solid |
| Example 47 | Formula (I-68) | 73 | 208-209 | Yellow solid |

Reference Example 1

Synthesis of 5-methyl-7-bromo 5H-indolo[3,2-b]quinoline (structure of which is shown in the following formula (II)).

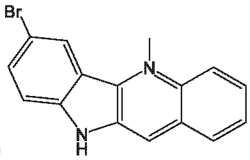

Formula (II)

2mmol $POCl_3$ (phosphorus oxychloride) was dripped to 1mL N,N-dimethylformamide at -16°C and stirred for 0.5h, and then

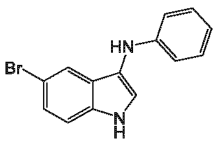

was added to the above solution at room temperature and stirred for 1h. Then saturated sodium bicarbonate solution was added to the reaction solution, the obtained reaction solution was extracted with ethyl acetate, organic layer of which was washed with saturated sodium bicarbonate solution, and the organic phase was combined, dried with magnesium sulfate and concentrated under reduced pressure to obtain solid residue. Using dichloromethane/methanol as eluent, the yellow solid product was purified by column chromatography.

0.1mol of dimethylamine hydrochloride was dissolved in 1mL N,N-dimethylformamide, then 0.05mol of the yellow solid product obtained in the previous step was added to the reaction system, the obtained reaction system was heated and refluxed for 1.5h, cooled after the reaction was completed, after adding 5mL of 5% sodium carbonate solution, the reaction system was stirred at room temperature for 10min, extracted with ethyl acetate for 3 times, and then washed with saturated sodium carbonate. The organic phase was combined, dried with magnesium sulfate and concentrated under reduced pressure to concentrate solid residue. The purple solid product was purified by column chromatography with methylene chloride/methanol as eluent, and the yield was 35%.

The results of structural characterization were as follows:

$^1$HNMR(400MHz,DMSO-d6)δ9.05(s,1H),8.70(s,1H),8.58(d,J=9.0Hz,1H),8.44(d,J=8.3Hz,1H),8.0 2-7.87(m,1H),7.74(t,J=7.5Hz, 1H),7.69-7.60(m,2H),4.92(s,3H).

Reference Example 2

Synthesis of 11-(4'-(2-aminoethyl)-piperazinyl) substituted cryptolepine iodide salt (structure of which is shown in the following formula (III)).

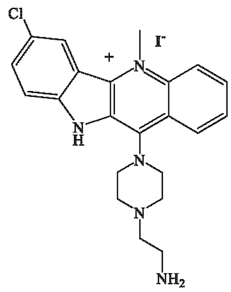

Formula (III)

0.1mol of 7-chloro-11-iodo-methyl indole [3,2-b] quinoline was mixed with 60mL of ethylene glycol diethyl ether, 0.15mol of 4-(2-aminoethyl) piperazine was added into the above solution and stirred at 120°C for 0.5h, the precipitated solids were cooled, the reddish-brown solids were collected by filtration, and yellow solids were obtained by recrystallization with the mixture of ethyl ether and ethanol, the yield was 80%.

The results of structural characterization were as follows:

$^1$HNMR(400Hz,DMSO-d6)δ8.60(d,J=8.4Hz,1H),8.44(s,1H),8.27(d,J=8.8Hz,1H),7.92(dd,J=8.4,7. 2Hz,1H),7.72(d,J=8.8Hz,1H),7.80(t,J=7.6Hz,1H),7.48(d,J=8.4Hz,1H,),4.55(s,3H),2.85(m,8H),2.65( m,4H).

Test Example 1

Determination of anti-tobacco Mosaic virus (TMV) activity by the following procedure:

1. Virus purification and concentration determination:

The virus crude extract was centrifuged with polyethylene glycol at twice and refrigerated at 4°C for use. The absorbance of 260nm wavelength was determined by ultraviolet spectrophotometer, and the virus concentration was calculated according to the formula.

$$\text{Virus concentration (mg/ml)}=(A_{260} \times \text{dilution ratio})/E^{0.1\%}{}_{1cm}{}^{260nm}.$$

Wherein E represents the extinction coefficient, that is, the light absorption (optical density) value of a suspension with a concentration of 0.1%(1mg/ml) at a wavelength of 260nm and an optical path of 1cm.

The $E^{0.1\%}{}_{1cm}{}^{260nm}$ of TMV is 3.1.

2. Preparation of cryptolepine salt derivative solution:

After weighing, cryptolepine salt derivative, ningnanmycin and the original drug of ribavirin were dissolved in DMF to obtain $1\times10^5$μg/mL mother solution, and then diluted to the required concentration with 1‰ Tween 80 aqueous solution.

3. Live protection function:

Select the 3-5 leaf stage of tobacco Xanthi nc with uniform growth, spray the whole plant, repeat each treatment 3 times, and set 1‰ Tween80 aqueous solution as control. 24h later, the leaf surface was coated with emery (500 mesh), the disease venom was dipped with a brush, and the whole leaf surface was gently rubbed twice along the direction of the branch pulse by using the brush, the leaf was supported by palm of hand, the virus concentration was 10μg/mL, and the leaf was washed by water after inoculation. After 3 days, the number of disease spots was recorded and the prevnetion and control effect was calculated.

4. Live treatment effect:

Select the 3-5 leaf stage of tobacco Xanthi nc with uniform growth, use a brush to vaccinate the virus in the whole leaf, the virus concentration is 10μg/mL, and the leaf was washed with running water after inoculation. After the leaves were dried, the whole plant was sprayed with test sample, repeated 3 times every treatment, and 1‰ Tween 80 aqueous solution was set as control. After 3 days, the number of disease spots was recorded and the prevnetion and control effect was calculated.

5. Living passivation activity test:

Select the 3-5 leaf stage of tobacco Xanthi nc with uniform growth, mix the test sample agent with the same volume of virus juice to passivate for 30min, friction inoculation, virus concentration 20μg/mL, the leaf was washed with running water after inoculation, repeat 3 times, set 1‰ Tween 80 aqueous solution as control. 3 days later, the number of disease spots was calculated.

Relative inhibition rate (%)=[(number of dead spots of control group - number of dead spots of test sampe group)/ number of dead spots of control group] × 100%.

Anti-tobacco Mosaic virus passivation activity test of all compounds was at a treatment dose of 500μg/mL. The positive control was the commercial anti-plant virus agent viriazole.

The test results of the anti-TMV activity of the cryptolepine salt derivatives with the structures shown in formulas (I-1) to (I-22) and (I-44) to (I-68), Ningnanmycin and ribazole were shown in Table 4 below.

Table 4

| Sample Number | Relative inhibition rate (%) | | |
|---|---|---|---|
| | Live protection function | Live treatment effect | Living passivation activity test |
| Treatment dose (mg/L) | 500 | 500 | 500 |
| Example 1 | 18.9±2.6 | 20.3±1.1 | 18.5±0.6 |

(continued)

| Sample Number | Relative inhibition rate (%) | | |
|---|---|---|---|
| | Live protection function | Live treatment effect | Living passivation activity test |
| Example 2 | 26.5±3.0 | 20.5±0.9 | 25.3±2.3 |
| Example 3 | 18.1±3.1 | 19.1±0.1 | 20.3±0.2 |
| Example 4 | 21.6±3.4 | 18.6±1.2 | 14.1±1.3 |
| Example 5 | 46.7±4.1 | 35.8±4.8 | 39.3±2.5 |
| Example 6 | 48.4±4.7 | 45.2±3.6 | 41.0±2.2 |
| Example 7 | 44.5±0.4 | 43.0±3.5 | 47.7±3.1 |
| Example 8 | 18.8±4.2 | 23.1±0.5 | 18.6±1.2 |
| Example 9 | 23.8±3.5 | 19.1±0.1 | 23.1±0.9 |
| Example 10 | 17.0±1.0 | 10.2±0.7 | 19.1±0.2 |
| Example 11 | 27.6±1.4 | 30.1±1.1 | 22.3±1.0 |
| Example 12 | 23.4±3.3 | 19.1±1.0 | 20.3±2.1 |
| Example 13 | 50.8±1.0 | 52.0±2.4 | 46.1±3.9 |
| Example 14 | 15.3±1.2 | 18.9±1.2 | 20.1±1.5 |
| Example 15 | 15.6±0.4 | 22.1±0.2 | 20.0±1.0 |
| Example 16 | 28.1±4.5 | 30.1±1.1 | 18.9±0.8 |
| Example 17 | 30.2±1.7 | 23.5±2.1 | 33.1±1.1 |
| Example 18 | 45.6±0.9 | 38.7±2.4 | 40.8±2.9 |
| Example 19 | 47.0±2.8 | 42.4±4.9 | 44.2±3.4 |
| Example 20 | 51.3±3.5 | 48.5±3.0 | 53.0±2.1 |
| Example 21 | 41.4±3.9 | 32.3±1.8 | 35.9±4.0 |
| Example 22 | 14.0±1.1 | 18.2±1.1 | 15.5±1.1 |
| Example 23 | 33.0±2.9 | 30.1±1.0 | 35.2±1.1 |
| Example 24 | 36.9±3.7 | 30.3±0.8 | 35.2±1.0 |
| Example 25 | 27.3±3.0 | 22.1±1.1 | 35.1±0.1 |
| Example 26 | 43.1±3.4 | 45.4±1.9 | 37.0±3.0 |
| Example 27 | 52.1±2.8 | 46.3±4.2 | 47.9±1.5 |
| Example 28 | 20.5±1.7 | 19.6±0.2 | 25.3±1.2 |
| Example 29 | 38.6±4.6 | 36.5±2.3 | 30.5±0.9 |
| Example 30 | 20.9±2.9 | 21.5±1.1 | 30.0±0.8 |
| Example 31 | 29.5±1.3 | 28.9±1.1 | 30.5±0.2 |
| Example 32 | 25.1±4.3 | 30.1±0.4 | 28.7±2.1 |
| Example 33 | 57.9±4.6 | 51.7±3.6 | 49.8±3.1 |
| Example 34 | 50.3±2.1 | 43.9±2.0 | 41.2±4.6 |
| Example 35 | 41.0±2.4 | 48.0±1.5 | 44.3±2.9 |
| Example 36 | 29.8±2.1 | 30.1±0.2 | 28.7±1.1 |
| Example 37 | 31.6±3.2 | 20.7±1.2 | 33.6±0.8 |
| Example 38 | 35.0±4.1 | 40.2±1.0 | 30.7±1.2 |
| Example 39 | 25.4±3.8 | 30.1±0.2 | 28.9±2.1 |

(continued)

| Sample Number | Relative inhibition rate (%) | | |
|---|---|---|---|
| | Live protection function | Live treatment effect | Living passivation activity test |
| Example 40 | 45.8±3.8 | 39.5±3.1 | 38.6±2.5 |
| Example 41 | 40.8±4.7 | 34.1±2.3 | 30.6±4.0 |
| Example 42 | 28.1±3.0 | 30.2±2.1 | 25.1±0.2 |
| Example 43 | 26.1±1.0 | 28.3±0.2 | 30.0±2.1 |
| Example 44 | 27.3±4.4 | 33.1±0.3 | 35.3±2.1 |
| Embodiment 45 | 30.7±2.6 | 28.1±0.3 | 25.6±1.1 |
| Example 46 | 44.7±4.0 | 40.1±0.9 | 46.3±3.5 |
| Example 47 | 36.4±2.8 | 33.3±2.0 | 35.7±0.3 |
| Reference Example 1 | 15.1±2.0 | 10.5±3.0 | 10.2±1.8 |
| Reference Example 2 | 15.2±1.1 | 18.1±0.2 | 15.3±2.1 |
| Ningnanmycin | 58.3±4.1 | 56.9±2.8 | 59.0±2.2 |
| ribavirin | 39.5±2.0 | 36.1±3.7 | 38.0±1.3 |

As can be seen from the data in Table 4, at a dose of 500μg/mL, the vast majority of the examples showed better anti-TMV activity than reference example 1 and 2. Among them, the antiTMV activity of the cryptolepine salt derivative with the structures shown in formula (I-5), (I-6), (I-13), (I-20), (I-48), (I-54) and (I-55) was comparable to that of commercial Ningnanmycin. And formula (I-5), (I-6), (I-7), (I-13), (I-18), (I-19), (I-20), (I-21), (I-47), (I-48), (I-54), (I-55), (I-56), (I-61), (I-62) and (I-67) showed better TMV activity than the ribavirin.

Test Example 2

Bactericidal activity test, the determination procedure is as follows:
Test of resistance to rice sheath blight: Select uniformly growing rice seedlings and spray them on the leaves at the set concentration, set a water sprayring group as blank control, every treatment was repeated twice. After 24h, the rice seedlings were inoculated with rice sheath blight strains and placed at room temperature (25±4°C) for normal management after inoculation. The prevention and control effect was investigated by visual inspection 6 days after inoculation.
The results of bactericidal activity test of cryptolepine salt derivative with structures shown in formulas (I-1) to (I-22) and (I-44) to (I-68) and thifluzamide were shown in Table 5 below:

Table 5

| Samples | Resistance to rice sheath blight (%)/400mg/L |
|---|---|
| Example 1 | 95±5 |
| Example 2 | 40±0 |
| Example 3 | 35±5 |
| Example 4 | 60±0 |
| Example 5 | 55±5 |
| Example 6 | 40±0 |
| Example 7 | 40±0 |
| Example 8 | 30±0 |
| Example 9 | 70±0 |
| Example 10 | 30±0 |
| Example 11 | 40±0 |
| Example 12 | 40±0 |

(continued)

| Samples | Resistance to rice sheath blight (%)/400mg/L |
|---|---|
| Example 13 | 35±5 |
| Example 14 | 60±0 |
| Example 15 | 40±0 |
| Example 16 | 95±5 |
| Example 17 | 40±0 |
| Example 18 | 45±5 |
| Example 19 | 20±0 |
| Example 20 | 55±5 |
| Example 21 | 40±0 |
| Example 22 | 45±5 |
| Example 23 | 30±0 |
| Example 24 | 30±0 |
| Example 25 | 20±0 |
| Example 26 | 35±5 |
| Example 27 | 30±0 |
| Example 28 | 60±0 |
| Example 29 | 30±0 |
| Example 30 | 35±5 |
| Example 31 | 25±5 |
| Example 32 | 30±0 |
| Example 33 | 40±0 |
| Examples 34 | 20±0 |
| Example 35 | 25±5 |
| Example 36 | 35±5 |
| Example 37 | 40±0 |
| Example 38 | 30±0 |
| Example 39 | 25±5 |
| Example 40 | 20±0 |
| Example 41 | 40±0 |
| Example 42 | 25±5 |
| Example 43 | 30±0 |
| Example 44 | 55±5 |
| Example 45 | 30±0 |
| Example 46 | 30±0 |
| Example 47 | 45±5 |
| Reference Example 1 | 0 |
| Reference Example 2 | 0 |
| Thifluzamide | 100 |

From the data in Table 5, it can be seen that in the live pot test, the cryptolepine salt derivative showed certain

bactericidal activity against rice sheath blight, but reference examples 1 and 2 had no activity. Among them, the cryptolepine salt derivative shown in formula (I-1) and formula (I-16) showed more than 95% bactericidal activity against rice sheath blight. And the the cryptolepine salt derivative shown in formula (I-4), (I-5), (I-9), (I-14), (I-18), (I-20), (I-22), (I-49), (I-65) and (I-68) showed more than 50% bactericidal activity against rice sheath blight.

The product obtained by exmaples 1-47 was characterized to prove the structure correct, and the results are shown in Table 6.

Table 6

| Example serial number | Corresponding compound | Characterization result |
|---|---|---|
| Example 1 | formula (I-1) 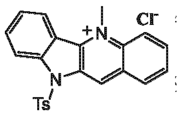 I-1 | $^1$HNMR(400MHz,DMSO-d6)δ9.98(d,J=10.7Hz,1H),8.96-8.84(m,3H),8.56-8.46(m,1H),8.3 4-8.27(m,1H),8.16-8.04(m,4H),7.81-7.75(m,1H),7.39(d,J=8.4Hz,2H),5.03(s,3H),2.27(s,3H)。 13CNMR(100MHz,DMSO-d6)δ147.5,142.1,137.6,136.0,135.2,133.0,131.7,131.6,131.1,12 9.1,128.3,127.7,127.4,126.3,118.9,118.2,115.2,41.7,21.5 。 HRMS(ESI),calculated for $C_{23}H_{19}N_2O_2S^+$[M-Cl]$^+$387.1162,found 387.1160。 |
| Example 2 | formula (I-2) 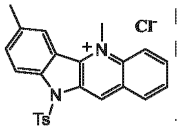 I-2 | $^1$HNMR(400MHz,DMSO-d6)δ9.98(s,1H),8.84(d,J=8.8Hz,2H),8.71(s,1H),8.46(d,J=8.7Hz,1 H),8.34-8.25(m,1H),8.12-8.01(m,3H),7.95(d,J=8.8Hz,1H),7.37(d,J=8.1Hz,2H),4.96(s,3H),2.60(s,3 H),2.27(s,3H)。 $^{13}$CNMR(100MHz,DMSO-d6)δ147.4,142.3,140.6,137.6,137.2,136.2,135.1,133.0,132.0,131 7,131.0,129.1,129.0,128.5,127.7,127.4,126.0,118.8,118.5,115.1,41.5,21.5,21.2。 HRMS(ESI),calculated for $C_{24}H_{21}N_2O_2S^+$[M-Cl]$^+$401.1318,found401.1315。 |
| Example 3 | formula (I-3) 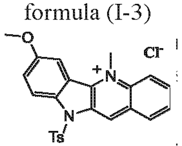 I-3 | $^1$HNMR(400MHz,DMSO-d6) δ9.94(s,1H),8.91-8.82(m,2H),8.42(d,J=9.3Hz,1H),8.34-8.27(m,1H),8.17(s,1H),8.10-8.05(m,1H),8.00(d,J=8.3Hz,2H),7.74-7.68(m,1H),7.35(d,J=8.1Hz,2H),5.01(s,3H),4.02(-s,3H),2.26(s,3H)。 $^{13}$CNMR(100MHz,DMSO-d6)δ157.4,147.4,141.9,137.7,136.7,135.3,132.9,132.1,131.7,131 0,129.4,129.1,127.6,127.4,124.5,119.2,118.9,116.4,110.1,56.9,41.5,21.5。 HRMS(ESI),calculated for $C_{24}H_{21}N_2O_3S^+$[M-Cl]$^+$417.1267,found 417.1265。 |
| Example 4 | formula (I-4) 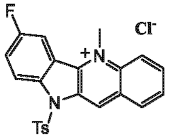 I-4 | $^1$HNMR(400MHz,DMSO-d6)δ10.04(s,1H),8.92-8.79(m,3H),8.60(dd,J=9.3,4.3Hz,1H),8.38 -8.28(m,1H),8.15-7.99(m,4H),7.39(d,J=8.2Hz,2H),4.97(s,3H),2.29(s,3H)。 $^{13}$CNMR(100MHz,DMSO-d6)δ147.6,141.7,138.6,137.7,135.6,132.9,132.3,131.7,131.1,129 8,129.3,127.7,123.9,123.6,119.4,118.9,117.1,114.2,114.0,41.4,21.5。 HRMS(ESI),calculated for $C_{23}H_{18}FN_2O_2S^+$[M-Cl]$^+$405.1068,found 405.1066。 |
| Example 5 | formula (I-5) 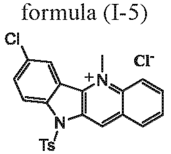 I-5 | $^1$HNMR(400MHz,DMSO-d6)δ10.05(s,1H),8.97(s,1H),8.87(d,J=8.2Hz,2H),8.59(d,J=9.1Hz, 1H),8.36-8.29(m,1H),8.21-8.04(m,4H),7.40(d,J=8.1Hz,2H),4.98(s,3H),2.29(s,3H)。 $^{13}$CNMR(100MHz,DMSO-d6)δ147.7,141.2,140.6,137.7,135.6,135.5,132.9,132.1,131.8,131 2,130.8,129.8,129.4,127.8,127.2,119.8,118.9,116.9,110.0,41.6,21.5。 HRMS(ESI),calculated for $C_{23}H_{18}ClN_2O_2S^+$[M-Cl]$^+$421.0772,found 421.0771。 |

(continued)

| Example serial number | Corresponding compound | Characterization result |
|---|---|---|
| Example 6 | formula (I-6) 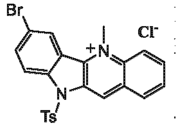 I-6 | $^{1}$HNMR(400MHz,DMSO-d6)δ10.04(s,1H),9.06(s,1H),8.87(d,J=8.2Hz,2H),8.52(d,J=9.0Hz, 1H),8.35-8.29(m,1H),8.27(d,J=9.1Hz,1H),8.14-8.07(m,3H),7.39(d,J=8.3Hz,2H),4.98(s,3H),2.29(s, 3H)。 $^{13}$CNMR(100MHz,DMSO-d6)δ147.7,141.1,141.0,138.3,137.7,135.6,132.9,131.9,131.7,131 2,130.0,129.7,129.4,127.8,120.2,118.9,118.8,117.1,41.7,21.5。 HRMS(ESI),calculated for $C_{23}H_{18}BrN_2O_2S^+[M-Cl]^+$465.0267,found 465.0269。 |
| Example 7 | formula (I-7) 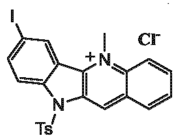 I-7 | $^{1}$HNMR(400MHz,CDCl3)δ8.79(s,1H),8.57-8.46(m,2H),8.07(t,J=7.8Hz,1H),7.96(d,J=8.0H z,2H),7.83(d,J=8.1Hz,1H),7.65(t,J=7.5Hz,1H),7.54(d,J=8.7Hz,1H),7.33(d,J=8.8Hz,1H),7.30-7.18( m,3H),4.96(s,3H),2.38(s,3H)。 $^{13}$CNMR(100MHz,CDCl3)δ144.4,142.9,141.6,140.0,135.2,134.7,133.4,132.7,132.5,129.7, 128.9,127.3,126.2,126.1,125.8,117.5,115.5,114.4,40.5,21.4 。 HRMS(ESI),calculated for $C_{23}H_{18}IN_2O_2S^+[M-Cl]^+$513.0128,found 513.0129。 |
| Example 8 | formula (I-8) 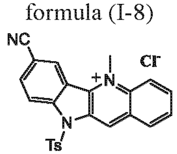 I-8 | $^{1}$HNMR(400MHz,CDCl3)δ8.07(d,J=8.6Hz,1H),7.88(s,1H),7.77(d,J=8.3Hz,2H),7.69(d,J=3. 7Hz,1H),7.59- 7.53(m,1H),7.28(s,1H),6.72(d,J=3.7Hz,1H),2.37(s,3H)。 $^{13}$CNMR(100MHz,CDCl3)δ145.8,136.4,134.8,130.7,130.2,128.4,127.6,126.9,126.4,119.3, 114.3,108.5,106.9,21.7。 HRMS(ESI),calculated for $C_{24}H_{18}N_3O_2S^+[M-Cl]^+$412.1114,found 412.1117。 |
| Example 9 | formula (I-9) 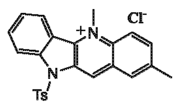 I-9 | $^{1}$HNMR(400MHz,DMSO-d6)δ9.88(s,1H),8.86(d,J=8.4Hz,1H),8.72(d,J=7.9Hz,1H),8.58(d,J =9.2Hz,2H),8.15(d,J=9.3Hz,1H),8.13-8.09(m,1H),8.07(d,J=8.0Hz,2H),7.81-7.74(m,1H),7.38(d,J=8 .1Hz,2H),4.94(s,3H),2.68(s,3H),2.28(s,3H)。 $^{13}$CNMR(100MHz,DMSO-d6) δ147.5,142.1,141.6,139.4,137.2,136.2,135.7,133.1,131.9,131 .7,131.1,130.1,130.0,128.3,128.1,127.7,127.7,126.3,118.5,118.5,115.4,41.4,21.5,21.3。 HRMS(ESI),calculated for $C_{24}H_{21}N_2O_2S^+[M-Cl]^+$401.1318,found 401.1318。 |
| Example 10 | formula (I-10) 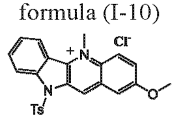 I-10 | $^{1}$HNMR(400MHz,DMSO-d6)δ9.86(s,1H),8.84(d,J=8.3Hz,1H),8.77(d,J=9.8Hz,1H),8.54(d,J =8.6Hz,1H),8.31(d,J=2.8Hz,1H),8.06(d,J=8.4Hz,3H),7.93-7.88(m,1H),7.78-7.72(m,1H),7.39(d,J=8 .3Hz,2H),4.95(s,3H),4.07(s,3H),2.29(s,3H)。 $^{13}$CNMR(100MHz,DMSO-d6)δ159.0,147.5,141.7,140.0,135.3,133.4,133.1,132.2,131.1,129 .6,127.8,127.6,127.4,127.2,126.2,120.5,118.5,115.3,109.2,56.8,41.7,21.5。 HRMS(ESI),calculated for $C_{24}H_{21}N_2O_3S^+[M-Cl]^+$417.1267,found 417.1268。 |

(continued)

| Example serial number | Corresponding compound | Characterization result |
|---|---|---|
| Example 11 | formula (I-11) 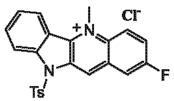 I-11 | $^1$HNMR(400MHz,DMSO-d6)δ10.00(s,1H),8.95(d,J=22.6Hz,2H),8.75(s,1H),8.57(d,J=7.1H z,1H),8.27(s,1H),8.10(s,3H),7.79(s,1H),7.41(s,2H),5.00(s,3H),2.29(s,3H)。 $^{13}$CNMR(100MHz,DMSO-d6)δ147.6,142.4,142.3,136.2,134.8,133.0,132.6,131.1,128.4,128 .2,127.8,126.4,124.8,124.5,122.4,118.2,115.4,114.9,114.7,42.1,21.5。 $^{19}$FNMR(376MHz,DMSO)δ-110.17--110.42(m)。 HRMS(ESI),calculated for $C_{23}H_{18}FN_2O_2S^+$[M-Cl]$^+$405.1068,found 405.1068。 |
| Example 12 | formula (I-12) 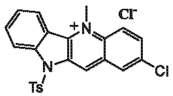 I-12 | $^1$HNMR(400MHz,DMSO-d6)δ9.98(s,1H),9.03(s,1H),8.95-8.86(m,2H),8.58(d,J=8.6Hz,1H), 8.32(d,J=9.6Hz,1H),8.15(t,J=8.0Hz,1H),8.10(d,J=8.0Hz,2H),7.79(t,J=7.8Hz,1H),7.40(d,J=8.1Hz,2 H),4.98(s,3H),2.29(s,3H)。 $^{13}$CNMR(100MHz,DMSO-d6)δ147.6,142.8,142.5,136.4,136.2,134.9,133.6,133.0,132.6,131 1,129.9,128.5,128.3,128.0,127.8,126.4,121.3,118.2,115.4,100.0,41.9,21.5。 HRMS(ESI),calculated for $C_{23}H_{18}ClN_2O_2S^+$[M-Cl]$^+$421.0772,found 421.0773。 |
| Example 13 | formula (I-13) 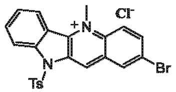 I-13 | $^1$HNMR(400MHz,DMSO-d6)δ9.97(s,1H),9.18(d,J=2.1Hz,1H),8.91(d,J=8.3Hz,1H),8.83(d,J =9.6Hz,1H),8.58(d,J=8.6Hz,1H),8.41(d,J=9.5Hz,1H),8.17-8.12(m,1H),8.10(d,J=8.4Hz,2H),7.82-7. 74(m,1H),7.39(d,J=8.3Hz,2H),4.97(s,3H),2.29(s,3H)。 $^{13}$CNMR(100MHz,DMSO-d6)δ147.6,142.7,142.5,137.4,136.5,136.4,133.2,133.0,132.4,131 1,128.7,128.5,127.9,127.8,126.4,122.2,121.2,118.2,115.4,41.9,21.5。 HRMS(ESI),calculated for $C_{23}H_{18}BrN_2O_2S^+$[M-Cl]$^+$465.0267,found 465.0268。 |
| Example 14 | formula (I-14) 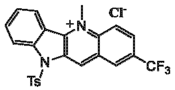 I-14 | $^1$HNMR(400MHz,DMSO-d6)δ10.23(s,1H),9.45(s,1H),9.09(d,J=9.4Hz,1H),8.97(d,J=8.3Hz, 1H),8.63(d,J=8.6Hz,1H),8.59-8.52(m,1H),8.19(t,J=8.0Hz,1H),8.14(d,J=8.5Hz,2H),7.82(t,J=7.7Hz, 1H),7.41(d,J=8.2Hz,2H),5.03(s,3H),2.30(s,3H)。 $^{13}$CNMR(100MHz,DMSO-d6)δ147.7,144.2,142.8,138.8,136.9,133.0,132.7,131.2,129.9,128 .8,127.8,126.7,126.5,121.0,118.1,115.4,42.0,21.5.19FNMR(376MHz,DMSO) δ-61.03(s)。 HRMS(ESI),calculated for $C_{24}H_{18}F_3N_2O_2S^+$[M-Cl]$^+$455.1036,found 455.1039。 |
| Example 15 | formula (I-15) 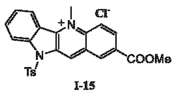 I-15 | $^1$HNMR(400MHz,DMSO-d6)δ10.21(s,1H),9.54(s,1H),8.94(t,J=7.8Hz,2H),8.63(d,J=8.4Hz, 2H),8.18(t,J=8.0Hz,3H),7.81(t,J=7.6Hz,1H),7.40(d,J=8.2Hz,2H),4.99(s,3H),4.04(s,3H),2.29(s,3H)。 $^{13}$CNMR(100MHz,DMSO-d6)δ165.3,147.7,143.7,142.8,139.2,136.7,133.9,133.3,133.1,132 3,131.1,130.2,129.4,128.6,127.8,127.0,126.4,119.8,118.1,115.4,53.5,41.9,21.5。 HRMS(ESI),calculated for $C_{25}H_{21}N_2O_4S^+$[M-Cl]$^+$445.1217,found 445.1216。 |
| Example 16 | formula (I-16) 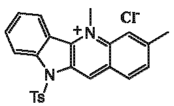 I-16 | $^1$HNMR(400MHz,DMSO-d6)δ9.95(s,1H),8.89(d,J=8.2Hz,1H),8.72(d,J=8.4Hz,1H),8.68(s,1 H),8.57(d,J=8.6Hz,1H),8.12(d,J=7.8Hz,1H),8.07(d,J=8.4Hz,2H),7.93(d,J=8.4Hz,1H),7.77(t,J=7.7H 2Hz,2H),4.94(s,3H),2.76(s,3H),2.28(s,3H)。 z,1H),7.38(d,J=8. $^{13}$CNMR(100MHz,DMSO-d6)δ147.5,147.1,142.0,141.7,137.9,135.6,133.1,131.2,131.1,129 1,128.1,127.7,126.2,125.9,118.5,117.7,115.4,41.3,22.8,21.5。 HRMS(ESI),calculated for $C_{24}H_{21}N_2O_2S^+$[M-Cl]$^+$401.1318,found 401.1318。 |

(continued)

| Example serial number | Corresponding compound | Characterization result |
|---|---|---|
| Example 17 | formula (I-17) 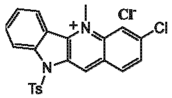 I-17 | $^{1}$HNMR(400MHz,DMSO-d6) δ10.05(s,1H),9.02(s,1H),8.96-8.85(m,2H),8.59(d,J=8.6Hz,1H ),8.20-8.13(m,2H),8.10(d,J=8.4Hz,2H),7.79(t,J=7.8Hz,1H),7.40(d,J=8.3Hz,2H),4.95(s,3H),2.29(s,3 H)。 $^{13}$CNMR(100MHz,DMSO-d6)δ147.1,142.4,142.0,139.8,137.6,135.9,132.9,132.6,131.5,130 6,129.3,128.6,127.9,127.3,125.9,125.7,117.9,117.7,114.9,41.3,21.0。 HRMS(ESI),calculated for $C_{23}H_{18}ClN_2O_2S^+$[M-Cl]$^+$421.0772,found 421.0773。 |
| Example 18 | formula (I-18) 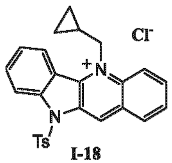 I-18 | $^{1}$HNMR(400MHz,DMSO-d6)δ10.06(s,1H),8.86(t,J=9.8Hz,2H),8.79(d,J=8.1Hz,1H),8.59(d, J=8.3Hz,1H),8.32(t,J=7.7Hz,1H),8.19-8.05(m,4H),7.84(t,J=7.3Hz,1H),7.41(d,J=7.3Hz,2H),5.52(d, J=4.9Hz,2H),2.30(s,3H),1.51(s,1H),0.82(s,2H),0.66(d,J=7.2Hz,2H)。 $^{13}$CNMR(100MHz,DMSO-d6)δ147.5,142.5,141.5,137.2,136.0,135.3,133.3,132.3,131.9,131 1,129.7,129.2,127.8,127.4,126.6,119.0,117.5,115.6,54.7,21.6,10.6,4.6。 HRMS(ESI),calculated for $C_{26}H_{23}N_2O_2S^+$[M-Cl]$^+$427.1475, found 427.1474。 |
| Example 19 | formula (I-19) 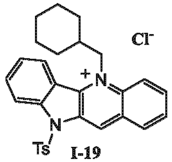 I-19 | $^{1}$HNMR(400MHz,DMSO-d6) δ10.07(s,1H),8.96(d,J=9.2Hz,1H),8.87(d,J=7.9Hz,1H),8.64-8 .56(m,2H),8.32-8.25(m,1H),8.17-8.06(m,4H),7.83(t,J=7.8Hz,1H),7.40(d,J=8.3Hz,2H),5.44(d,J=92. 5Hz,2H),2.89(s,1H),2.72(s,1H),2.51(s,1H),2.30(s,3H),2.09(s,1H),1.83(s,1H),1.62-1.53(m,2H),1.43-1.34(m,2H),1.19-1.04(m,2H)。 $^{13}$CNMR(100MHz,DMSO-d6)δ147.5,142.4,141.7,137.7,135.8,135.1,133.2,132.4,131.8,131 1,129.8,129.2,127.8,127.7,127.2,126.6,119.4,117.7,115.6,56.2,38.0,36.3,31.2,26.0,21.5。 HRMS(ESI),calculated for $C_{29}H_{29}N_2O_2S^+$[M-Cl]$^+$469.1944,found 469.1946。 |
| Example 20 | formula (I-20) 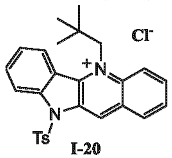 I-20 | $^{1}$HNMR(400MHz,DMSO-d6)δ10.12(s,1H),9.09(d,J=7.6Hz,1H),9.01-8.83(m,2H),8.56(d,J= 7.9Hz,1H),8.26(s,1H),8.11-7.94(m,4H),7.77(s,1H),7.35(d,J=7.0Hz,2H),5.77(s,1H),5.55(d,J=14.7H z,1H),2.27(s,3H),0.85(s,9H)。 $^{13}$CNMR(100MHz,DMSO-d6)δ147.5,142.9,142.4,138.7,135.9,134.5,132.9,132.6,131.7,131 0,129.3,128.2,127.9,127.7,126.2,120.7,118.6,115.9,59.0,37.4,28.6,21.5。 HRMS(ESI),calculated for $C_{27}H_{27}N_2O_2S^+$[M-Cl]$^+$443.1788,found 443.1790。 |
| Example 21 | formula (I-21) 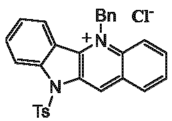 I-21 | $^{1}$HNMR(400MHz,CDCl3)δ9.87(s,1H),8.61(d,J=9.1Hz,1H),8.55(d,J=8.6Hz,1H),8.50(d,J=8. 1Hz,1H),8.29(d,J=8.3Hz,1H),8.17(t,J=7.5Hz,1H),7.99-7.92(m,2H),7.83(d,J=8.4Hz,2H),7.57(t,J=7. 8Hz,1H),7.33(d,J=5.2Hz,3H),7.31-7.26(m,6H),2.33(s,3H)。 $^{13}$CNMR(100MHz,CDCl3)δ147.5,143.3,141.9,137.7,136.0,133.0,132.1,131.9,130.8,130.7, 130.0,129.7,129.5,128.8,127.2,127.0,126.7,126.6,125.9,119.3,116.9,115.7,57.5,21.7。 HRMS(ESI),calculated for $C_{29}H_{23}N_2O_2S^+$[M-Cl]$^+$463.1475,found 463.1476。 |

(continued)

| Example serial number | Corresponding compound | Characterization result |
|---|---|---|
| Example 22 | formula (I-22) 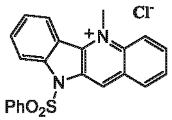 I-22 | $^{1}$HNMR(400MHz,DMSO-d6)δ10.03(s,1H),8.92(d,J=8.2Hz,1H),8.85(d,J=8.6Hz,2H),8.58(d J=8.5Hz,1H).8.31(t,J=7.6Hz,1H),8.21(d,J=7.7Hz,2H),8.16-8.07(m,2H),7.79(t,J=7.7Hz,1H),7.74(t, J=7.4Hz,1H),7.60(t,J=7.7Hz,2H),4.98(s,3H)。 $^{13}$CNMR(100MHz,DMSO-d6)δ142.4,142.2,137.6,136.4,136.0,135.2,131.8,131.7,130.7,129 2,129.1,128.4,127.7,127.5,126.4,118.8,118.4,115.4,110.0,41.5。 HRMS(ESI),calculated for $C_{22}H_{17}N_2O_2S^+$[M-Cl]$^+$373.1005,found 373.1004。 |
| Example 23 | formula (I-44) 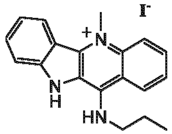 I-44 | $^{1}$HNMR(400MHz,DMSO-d6)δ11.64(s,1H),8.69(d,J=8.5Hz,2H),8.51(d,J= 8.4Hz,1H),8.30(d,J-8.9Hz,1H),8.04-7.96(m,1H),7.84(d,J=8.4Hz,1H),7.75-7.65(m,2H),7.41-7.31(m,1H),4.55(s,3H),4.04(t,J=6.4Hz,2H),1.94-1.79(m,2H),1.04(t,J=7.3Hz,3H)。 $^{13}$CNMR(100MHz,DMSO-d6)δ144.0,142.9,137.5,135.8,132.7,130.9,124.7,124.4,124.4,121 .2,117.8,116.6,115.5,114.8,113.9,47.4,38.5,23.5,11.7。 HRMS(ESI),calculated for $C_{19}H_{20}N_3^+$[M-I]$^+$290.1652,found 290.1651。 |
| Example 24 | formula (I-45) 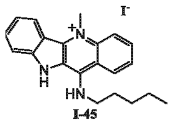 I-45 | $^{1}$HNMR(400MHz,DMSO-$d_6$)δ11.61(s,1H),8.69(d,J=7.7Hz,2H),8.50(d,J=7.9Hz,1H),8.29(d, J=8.5Hz,1H),8.06-7.95(m,1H),7.84(d,J=7.8Hz,1H),7.76-7.61(m,2H),7.42-7.28(m,1H),4.54(s,3H),4. 06(s,2H),1.93-1.69(m,2H),1.53-1.30(m,4H),0.90(t,J=6.4Hz,3H)。 $^{13}$CNMR(100MHz,DMSO-$d_6$)δ143.5,142.4,137.0,135.3,132.2,130.4,124.2,123.9,120.7,117 .3,116.1,115.0,114.3,113.4,45.4,38.0,29.3,28.4,21.9,13.9。 HRMS(ESI),calculated for $C_{21}H_{24}N_3^+$[M-I]$^+$318.1965,found 318.1964。 |
| Example 25 | formula (I-46) 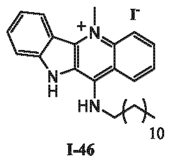 I-46 | $^{1}$HNMR(400MHz,DMSO-$d_6$)δ11.62(s,1H),8.69(d,J=7.8Hz,2H),8.51(d,J=8.1Hz,1H),8.31(d, J=8.5Hz,1H),8.05-7.96(m,1H),7.84(d,J=7.9Hz,1H),7.75-7.64(m,2H),7.41-7.30(m,1H) 4.55(s,3H) 4. 07(s,2H),1.44(s,2H),1.24(d,J=52.3Hz,18H),0.82(t,J=6.3Hz,3H)。 $^{13}$CNMR(100MHz,DMSO-$d_6$)δ144.0,143.0,137.6,135.8,132.7,130.9,124.7,124.4,121.2,117 .8,116.6,115.6,114.9,113.9,45.9,38.5,31.7,30.1,29.5,29.4,29.2,26.7,22.5,14.4 。 HRMS(ESI),calculated for $C_{28}H_{38}N_3^+$[M-I]$^+$416.3060,found 416.3060。 |
| Example 26 | formula (I-47) 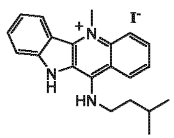 I-47 | $^{1}$HNMR(400MHz,MeOH-$d_4$) δ8.57(d,J=8.6Hz,1H),8.49(d,J=8.5Hz,1H),8.24(d,J=8.9Hz,1H),8.04-7.97(m,1H),7.83(d,J=8.4Hz,1H),7.75-7.66(m,2H),7.45-7.37(m,1H),4.61(s,3H),4.17(t,J=7.1Hz,2-H),1.89-1.78(m,3H),1.03(d,J=6.3Hz,6H)。 $^{13}$CNMR(100MHz,MeOH-$d_4$)δ144.1,143.1,137.7,136.1,132.3,130.7,124.2,123.7,123.3,121.0,116.7, 115.6,114.8,113.1,110.0,44.1,38.7,37.3,25.6,21.5。 HRMS(ESI),calculated for $C_{21}H_{24}N_3^+$[M-I]$^+$318.1965,found 318.1966。 |

(continued)

| Example serial number | Corresponding compound | Characterization result |
|---|---|---|
| Example 27 | formula (I-48) 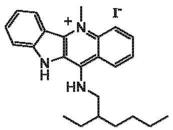 I-48 | $^{1}$HNMR(400MHz,DMSO-$d_6$)δ11.75(s,1H),8.75(d,J=8.1Hz,1H),8.59(d,J=8.5Hz,2H),8.38(d,J=8.8 Hz,1H),8.09-7.99(m,1H),7.85(d,J=8.4Hz,1H),7.81-7.68(m,2H),7.46-7.35(m,1H),4.63 (s,3H),4.02(s,2H),1 .94-1.85(m,1H),1.52-1.33(m,4H),1.32-1.19(m,4H),0.87(t,J=7.4Hz,3H),0.82(t,J=7.1Hz,3H)。 $^{13}$CNMR(100MHz,DMSO-$d_6$)δ144.4,143.1,137.7,136.3,132.8,131.2,125.0,124.6,124.3,121.4,118.0, 117.3,115.9,115.3,114.0,49.6,38.6,30.5,28.5,23.9,22.9,14.3,10.9。 HRMS(ESI),calculated for $C_{24}H_{30}N_3^+$[M-I]$^+$360.2434,found 360.2433。 |
| Example 28 | formula (I-49) 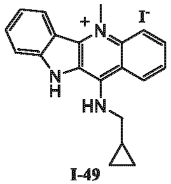 I-49 | $^{1}$HNMR(400MHz,DMSO-$d_6$)δ11.70(s,1H),8.87(s,1H),8.73(d,J=8.5Hz,1H),8.52(d,J=8.4Hz, 1H),8.32(d,J=8.9Hz,1H),8.05-7.97(m,1H),7.85(d,J=8.3Hz,1H),7.74-7.66(m,2H),7.40-7.32(m,1H),4 .57(s,3H),3.97(d,J=6.7Hz,2H),1.38-1.26(m,1H),0.63-0.54(m,2-H),0.52-0.42(m,2H)。 $^{13}$CNMR(100MHz,DMSO-$d_6$)δ143.9,143.0,137.6,135.8,132.8,131.0,124.7,124.5,124.4,121 .2,117.8,116.7,115.6,114.9,114.0,50.2,38.5,12.0,4.3。 HRMS(ESI),calculated for $C_{20}H_{20}N_3^+$[M-I]$^+$302.1652,found 302.1650。 |
| Example 29 | formula (I-50) 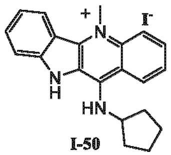 I-50 | $^{1}$HNMR(400MHz,DMSO-$d_6$)δ11.63(s,1H),8.76(d,J=8.4Hz,1H),8.55(d,J=8.3Hz,1H),8.46-8. 29(m,2H),8.06-7.97(m,1H),7.88(d,J=8.3Hz,1H),7.78-7.66(m,2H),7.44-7.33(m,1H),5.02(s,1H),4.60 (s,3H),2.29-2.14(m,2H),1.98-1.89(m,2H),1.88-1.78(m,2H),1.73(d,J=6.1Hz,2H)。 $^{13}$CNMR(100MHz,DMSO-$d_6$)δ143.5,143.0,137.6,136.0,132.8,131.1,124.9,124.8,124.3,121 .2,117.8,116.8,115.7,114.9,114.0,57.0,38.6,34.2,24.5。 HRMS(ESI),calculated for $C_{21}H_{22}N_3^+$[M-I]$^+$316.1808,found 316.1808。 |
| Example 30 | formula (I-51) 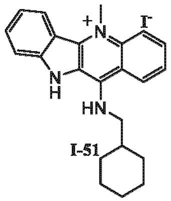 I-51 | $^{1}$HNMR(400MHz,DMSO-$d_6$)δ11.86(s,1H),8.77(d,J=8.5Hz,2H),8.56(d,J=8.4Hz,1H),8.35(d, J=8.9Hz,1H),8.02(t,J=7.8Hz,1H),7.88(d,J=8.4Hz,1H),7.79-7.67(m,2H),7.39(t,J=7.6Hz,1H),4.61(s, 3H),3.98(s,2H),1.85(d,J=10.3Hz,3H),1.64(d,J=23.4Hz,3H),1.23-1.11(m,3H),1.10-0.99(m,2H)。 $^{13}$CNMR(100MHz,DMSO-$d_6$)δ144.3,143.0,137.7,136.0,132.8,131.0,124.8,124.5,124.5,121 .3,118.0,117.1,115.8,115.1,114.1,54.8,52.0,38.5,30.7,26.4,25.8 。 HRMS(ESI),calculated for $C_{23}H_{26}N_3^+$[M-I]$^+$344.2121,found 344.2122。 |

(continued)

| Example serial number | Corresponding compound | Characterization result |
|---|---|---|
| Example 31 | formula (I-52) 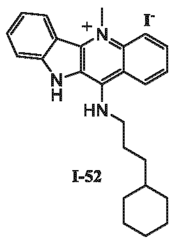 I-52 | $^1$HNMR(400MHz,DMSO-$d_6$)δ11.87(s,1H),8.88(s,1H),8.73(d,J=7.5Hz,1H),8.53(d,J=7.4Hz, 1H),8.32(d,J=8.1Hz,1H),8.05-7.97(m,1H),7.87(d,J=7.3Hz,1H),7.76-7.65(m,2H),7.37(t,J=6.2Hz,1H ),4.58(s,3H),4.09(s,2H),1.82(s,2H),1.73-1.50(m,6H),1.35(d,J=5.1Hz,2-H),1.21-1.10(m,3H),0.91-0.8 0(m,2H)。 $^{13}$CNMR(100MHz,DMSO-$d_6$)δ144.1,143.0,137.7,135.7,132.7,130.9,124.7,124.6,124.3,121 .2,117.8,116.8,115.6,115.0,114.0,46.2,38.5,37.2,34.4,33.2,27.6,26.6,26.2 。 HRMS(ESI),calculated for $C_{25}H_{30}N_3^+$[M-I]$^+$372.2434,found 372.2437。 |
| Example 32 | formula (I-53) 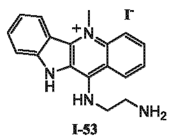 I-53 | $^1$HNMR(400MHz,DMSO-$d_6$) δ8.60(d,J=8.4Hz,1H),8.42(d,J=8.4Hz,1H),8.23(d,J=8.9Hz,1H ),7.95-7.89(m,1H),7.72(d,J=8.4Hz,1H),7.63-7.55(m,2H),7.23-7.18(m,1H),4.51(s,3H),4.28-4.21(m, 2H),3.20(s,2H)。 $^{13}$CNMR(100MHz,DMSO-$d_6$)δ145.6,145.0,137.1,135.2,131.8,129.5,124.2,124.1,123.4,120 .8,119.7,117.3,115.4,115.4,46.6,40.8,38.0 。 HRMS(ESI),calculated for $C_{18}H_{19}N_4^+$[M-I]$^+$291.1604,found 291.1602。 |
| Example 33 | formula (I-54) 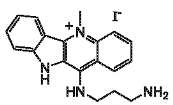 I-54 | $^1$HNMR(400MHz,DMSO-$d_6$) δ8.62(d,J=8.2Hz,1H),8.41(d,J=8.2Hz,1H),8.25(d,J=8.8Hz,1H ),7.91(t,J=7.4Hz,1H),7.67(d,J=8.3Hz,1H),7.59(t,J=7.1Hz,1H),7.50(t,J=7.0Hz,1H),7.14-7.09(m,1H), 4.57(s,3H),4.52(s,2H),3.12(s,1H),2.93(s,2H),2.09(s,2H),1.61(s,2H)。 $^{13}$CNMR(100MHz,DMSO-$d_6$)δ149.5,148.8,145.2,136.6,135.4,131.2,128.2,124.3,124.0,122.7,118.1,117.1,116.9,115.3,114.5,42.1,38.6,37.9,30.2。 HRMS(ESI),calculated for $C_{19}H_{21}N_4^+$[M-I]$^+$305.1761,found 305.1759。 |
| Example 34 | formula (I-55) 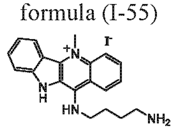 I-55 | $^1$HNMR(400MHz,DMSO-$d_6$) δ8.69(d,J=8.5Hz,1H),8.51(d,J=8.4Hz,1H),8.31(d,J=8.9Hz,1H ),8.01-7.95(m,1H),7.81(d,J=8.5Hz,1H),7.71-7.61(m,2H),7.28(t,J=7.6Hz,1H),4.59(s,3H),4.23(t,J=6. 7Hz,2H),2.90(t,J=7.1Hz,2H),1.94-1.83(m,2H),1.78-1.66(m,2H)。 $^{13}$CNMR(100MHz,DMSO-$d_6$)δ145.6,144.2,137.3,135.7,132.2,129.9,124.5,124.3,123.7,120 .0,119.6,117.6,115.3,115.2,115.0,44.7,39.1,38.3,27.4,24.9 。 HRMS(ESI),calculated for $C_{20}H_{23}N_4^+$[M-I]$^+$319.1917,found 319.1918。 |
| Example 35 | formula (I-56) 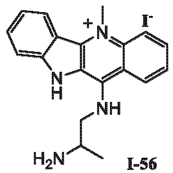 I-56 | $^1$HNMR(400MHz,DMSO-$d_6$)δ8.64(d,J=8.3Hz,1H),8.41(d,J=8.5Hz,1H),8.21(d,J=8.9Hz,1H ),7.91(t,1H),7.71(d,J=8.4Hz,1H),7.63 -7.53(m,2H),7.19(t,1H),4.51(s,3H),3.50(t,J=10.6,6.6Hz,1H),1 .85(s,2H),1.33(d,J=6.5Hz,3H)。 $^{13}$CNMR(100MHz,DMSO-$d_6$)δ170.2,145.2,144.8,136.8,134.5,131.3,128.8,123.8,123.7,122 .8,120.7,119.1,116.7,115.3,115.0,46.7,37.5,22.6,17.2 。 HRMS(ESI),calculated for $C_{19}H_{21}N_4^+$[M-I]$^+$305.1761,found 305.1760。 |

(continued)

| Example serial number | Corresponding compound | Characterization result |
|---|---|---|
| Example 36 | formula (I-57)<br>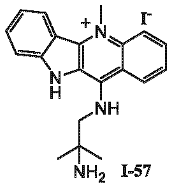<br>I-57 | $^{1}$HNMR(400MHz,DMSO-$d_6$)<br>δ8.73(d,J=8.4Hz,1H),8.40(d,J=8.4Hz,1H),8.15(d,J=8.8Hz,1H ),7.91-7.85(m,1H),7.69(d,J=8.4Hz,1H),7.59-7.50(m,2H),7.21-7.15(m,1H),4.49(s,3H),4.14(s,2H),1. 88(s,1H),1.41(s,6H),1.12(s,2H)。<br>$^{13}$CNMR(100MHz,DMSO-$d_6$)δ146.2,137.6,131.4,128.5,124.8,123.9,122.7,122.6,119.2,116 .9,116.8,115.8,52.7,37.7,26.7,24.9。<br>HRMS(ESI),calculated for $C_{20}H_{23}N_4^+$[M-I]$^+$319.1917,found 319.1917。 |
| Example 37 | formula (I-58)<br>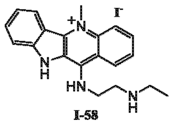<br>I-58 | $^{1}$HNMR(400MHz,MeOH-$d_4$)<br>δ8.50(d,J=8.5Hz,1H),8.41(d,J=8.5Hz,1H),8.19(d,J=8.9Hz,1H) ,7.99-7.94(m,1H),7.76(d,J=8.4Hz,1H),7.68-7.62(m,2H),7.37-7.31(m,1H),4.54(s,3H),4.24-4.17(m,2 H),3.23-3.19(m,2H),2.83(q,J=7.2Hz,2H),1.16(t,J=7.2Hz,3H)。<br>$^{13}$CNMR(100MHz,MeOH-$d_4$)δ143.4,137.4,136.1,132.1,130.5,124.9,124.1,123.6,123.1,120. 7,116.7,115.5,115.2,113.3,45.2,43.7,37.2,13.2。<br>HRMS(ESI),calculated for $C_{20}H_{23}N_4^+$[M-I]$^+$319.1917,found 319.1917。 |
| Example 38 | formula (I-59)<br>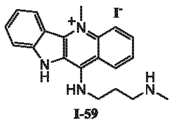<br>I-59 | $^{1}$HNMR(400MHz,MeOH-$d_4$)<br>δ8.48(d,J=8.4Hz,1H),8.38(d,J=8.5Hz,1H),8.16(d,J=8.9Hz,1H) ,8.00-7.92(m,1H),7.75(d,J=8.4Hz,1H),7.70-7.60(m,2H),7.32(t,J=7.7Hz,1H),4.49(s,3H),4.20(t,J=6.3 Hz,2H),2.87(t,J=6.0Hz,2H),2.47(s,3H),2.20-2.09(m,2H)。<br>$^{13}$CNMR(100MHz,MeOH-$d_4$)δ144.5,144.0,137.3,136.1,132.0,130.3,124.0,123.5,123.0,120. 5,116.6,115.3,114.9,113.4,100.0,46.8,42.9,37.1,34.0,28.7。<br>HRMS(ESI),calculated for $C_{20}H_{23}N_4^+$[M-I]$^+$319.1917,found 319.1917。 |
| Example 39 | formula (I-60)<br>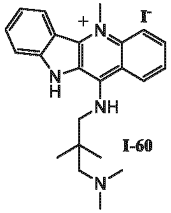<br>I-60 | $^{1}$HNMR(400MHz,DMSO-$d_6$)δ10.76(s,1H),8.57(d,J=8.4Hz,1H),8.36(d,J=8.9Hz,1H),8.19(d,J=8.4 Hz,1H),8.07-8.00(m,1H),7.86-7.71(m,3H),7.42-7.34(m,1H),4.60(s,3H),4.15(s,2H),3.36(s,1H), 2.62(s,2H), 2.46(s,6H),1.15(s,6H) 。<br>$^{13}$CNMR(100MHz,DMSO-$d_6$)δ144.5,143.0,137.7,135.8,132.8,130.9,125.0,124.8,123.0,121.2,118.1, 116.7,115.6,115.0,114.0,100.0,69.9,57.3,48.2,38.4,35.4,25.5。<br>HRMS(ESI),calculated for $C_{23}H_{29}N_4^+$[M-I]$^+$361.2387,found 361.2385。 |
| Example 40 | formula (I-61)<br>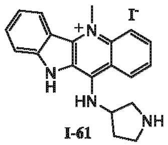<br>I-61 | $^{1}$HNMR(400MHz,DMSO-$d_6$)δ8.42(d,J=8.3Hz,2H),8.14(d,J=8.7Hz,1H),7.94-7.88(m,1H),7. 81(d,J=8.2Hz,1H),7.66-7.60(m,1H),7.55-7.51(m,1H),7.34-7.26(m,1H),4.46(s,3H),3.91(d,J=10.2Hz ,1H),3.68(s,1H),2.15(s,1H),1.91(d,J=5.2Hz,2H),1.21(s,1H),0.85(s,1H),<br>$^{13}$CNMR(100MHz,DMSO-$d_6$)δ147.5,142.5,139.1,134.4,132.2,129.9,128.1,124.1,122.0,120 .8,119.6,116.8,116.5,114.7,114.2,63.9,53.8,50.8,38.5,33.8。<br>HRMS(ESI),calculated for $C_{20}H_{21}N_4^+$[M-I]$^+$317.1761,found 317.1761。 |

(continued)

| Example serial number | Corresponding compound | Characterization result |
|---|---|---|
| Example 41 | formula (I-62) 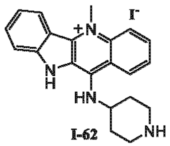 I-62 | $^1$HNMR(400MHz,DMSO-$d_6$) δ8.74(d,J=8.3Hz,1H),8.52(d,J=7.9Hz,1H),8.33(d,J=8.8Hz,1H ),8.00(t,1H),7.86(d,J=8.3Hz,1H),7.73-7.62(m,2H),7.31(t,1H),5.05(s,1H),4.60(s,3H),3.49-3.39(m,1 H),3.33(d,J=11.9Hz,2H),3.10(t,J=11.7Hz,2H),2.22-2.13(m,2H),1.96-1.88(m,2H)。 HRMS(ESI),calculated for $C_{21}H_{23}N_4^+$[M-I]$^+$331.1917,found 331.1919。 |
| Example 42 | formula (I-63) 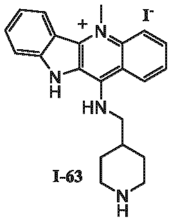 I-63 | $^1$HNMR(400MHz,DMSO-$d_6$) δ8.77(d,J=8.5Hz,1H),8.53(d,J=8.4Hz,1H),8.33(d,J=8.9Hz,1H),8.03-7.97(m,1H),7.85(d,J=8.4Hz,1H),7.68(t,J=6.9Hz,2H),7.32(t,J=7.6Hz,1H),4.60(s,3H),4.15(d,J =6.8Hz,2H),3.24(d,J=12.4Hz,2H),2.76(t,J=11.7Hz,2H),2.13(s,1H),1.95(d,J=12.3Hz,2H),1.53-1.40( m,2H)。 $^{13}$CNMR(100MHz,DMSO-$d_6$)δ144.3,137.5,135.8,132.4,130.3,124.6,124.5,124.1,120.6,118 8,118.2,116.6,115.7,115.1,114.9,50.4,43.6,38.4,35.1,27.1。 HRMS(ESI),calculated for $C_{22}H_{25}N_4^+$[M-I]$^+$345.2074,found 345.2073。 |
| Example 43 | formula (I-64) 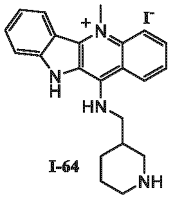 I-64 | $^1$HNMR(400MHz,DMSO-$d_6$)δ8.92(s,1H),8.72(d,J=8.4Hz,1H),8.54(d,J=8.4Hz,1H),8.35(d,J =8.9Hz,1H),8.07-7.98(m,1H),7.88(d,J=8.3Hz,1H),7.79-7.67(m,2H),7.43-7.33(m,1H),4.60(s,3H),4. 20(s,1H),4.11-3.90(m,2H),3.18(d,J=11.9Hz,2H),2.92-2.79(m,2H),2.32(s,1H),2.00-1.79(m,2H),1.61 -1.34(m,2H)。 $^{13}$CNMR(100MHz,DMSO-$d_6$)δ144.1,137.5,136.2,132.7,132.7,131.0,124.8,124.5,124.4,121 1,117.9,115.7,115.0,114.4,100.0,47.9,46.3,44.0,38.6,34.5,26.1 。 HRMS(ESI),calculated for $C_{22}H_{25}N_4^+$[M-I]$^+$345.2074,found 345.2074。 |
| Example 44 | formula (I-65) 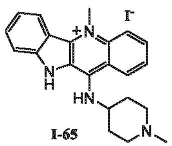 I-65 | $^1$HNMR(400MHz,DMSO-$d_6$)δ11.90(s,1H),8.74(d,J=8.1Hz,1H),8.55(d,J=8.1Hz,1H),8.36(d,J=8.3 Hz,2H),8.04(t,J=7.5Hz,1H),7.93(d,J=8.1Hz,1H),7.81-7.68(m,2H),7.39(t,J=7.2Hz,1H),4.86(s,1H),4.62 (s, 3H),3.28-3.15(m,4H),2.77(s,3H),2.29-2.08(m,4H)。 $^{13}$CNMR(100MHz,DMSO-$d_6$)δ143.4,143.3,137.5,136.7,132.9,131.4,125.0,124.6,121.5,117.9,117.4, 116.2,115.2,114.1,54.9,52.6,50.1,38.8,30.4。 HRMS(ESI),calculated for $C_{22}H_{25}N_4^+$[M-I]$^+$345.2074,found 345.2074。 |

(continued)

| Example serial number | Corresponding compound | Characterization result |
|---|---|---|
| Example 45 | formula (I-66) 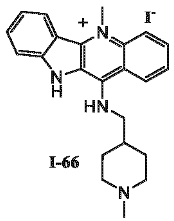 I-66 | $^1$HNMR(400MHz,DMSO-$d_6$)δ11.83(s,1H),8.86(s,1H),8.77(d,J=8.6Hz,1H),8.60(d,J=8.6 Hz,1H),8.39(d,J=9.0Hz,1H),8.05(t,1H),7.89(d,J=8.4Hz,1H),7.81-7.72(m,2H),7.41(t,J=7.7Hz,1H),4.65 (s,3H),4.07( s,2H),3.11(s,5H),2.70(s,3H),2.16-1.96(m,3H),1.63-1.47(m,2H)。 $^{13}$CNMR(100MHz,DMSO-$d_6$)δ144.1,143.3,137.7,136.3,132.9,131.2,124.9,124.7,124.4,121.4,118.0, 117.0,115.8,115.1,114.1,54.9,49.9,43.1,38.6,34.0,27.2, HRMS(ESI),calculated for $C_{23}H_{27}N_4^+$[M-I]$^+$359.2230,found 359.2229。 |
| Example 46 | formula (I-67) 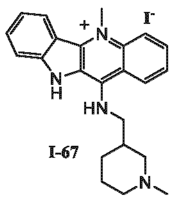 I-67 | $^1$HNMR(400MHz,DMSO-$d_6$) δ8.68(d,J=8.5Hz,1H),8.53(d,J=8.4Hz,1H),8.32(d,J=8.9Hz,1H ),8.00(t,1H),7.79(d,J=8.3Hz,1H),7.70(t,J=7.6Hz,2H),7.35(t,J=7.6Hz,1H),4.57(s,3H),4.50(s,1H),3.69(d,J=15.0Hz,1H),2.47-2.33(m,2H),2.31-2.22(m,1H),2.08(s,3H),1.86-1.78(m,1H),1.76-1.67(m,1H) ,1.66-1.56(m,1H),1.54-1.43(m,1H),1.27-1.18(m,1H),0.88-0.79(m,1H)。 $^{13}$CNMR(100MHz,DMSO-$d_6$)δ144.8,143.9,137.5,136.3,132.6,130.8,124.7,124.4,124.1,120 9,117.8,115.6,115.5,114.4,55.5,47.8,45.8,38.4,35.1,26.3,22.4,14.4。 HRMS(ESI),calculated for $C_{23}H_{27}N_4^+$[M-I]$^+$359.2230,found 359.2231。 |
| Example 47 | formula (I-68) 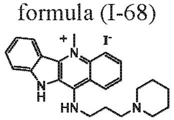 I-68 | $^1$HNMR(400MHz,DMSO-$d_6$)δ8.89(s,1H),8.65(d,J=8.3Hz,1H),8.53(d,J=8.2Hz,1H),8.33(d,J =8.8Hz,1H),8.06-7.98(m,1H),7.88(d,J=8.2Hz,1H),7.77-7.67(m,2H),7.42-7.33(m,1H),4.56(s,3H),4. 18(s,2H),3.14-2.78(m,6H),2.17(s,2H),1.67(s,4H),1.52(s,2H)。 $^{13}$CNMR(100MHz,DMSO-$d_6$)δ143.4,142.7,137.0,135.5,132.3,130.6,124.3,124.0,123.9,120 .8,117.4,116.2,115.1,114.4,113.5,54.2,52.7,43.1,38.1,24.7,23.3,21.9。 HRMS(ESI),calculated for $C_{24}H_{29}N_4^+$[M-I]$^+$373.2387,found 373.2383。 |

The preferred embodiments of the invention are described in detail above, however, the invention is not limited to this. Within the scope of the technical conception of the invention, a variety of simple variants of the technical scheme of the invention can be carried out, including the combination of each technical feature in any other suitable way. These simple variants and combinations shall also be regarded as the contents disclosed by the invention and belong to the scope of protection of the invention.

## Claims

1. A cryptolepine salt derivative with a chemical structure shown in formula (I),

formula (I) 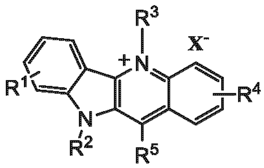 ;

wherein

$R^1$ is selected from at least one group consisting of hydrogen, C1-C6 alkyl, C1-C6 alkoxy, halogen, and cyano;
$R^2$ is selected from at least one group consisting of hydrogen and protecting group;
$R^3$ is selected from at least one group consisting of substituted or unsubstituted C1-C6 alkyl, and C6-C20 aromatic group; wherein substituent of substituted C1-C6 alkyl is selected from at least one group consisting of C3-C6 cycloalkyl;
$R^4$ is selected from at least one group consisting of hydrogen, C1-C6 alkyl, C1-C6 alkoxy, halogen, C1-C6 fluoroalkyl, and C2-C5 ester group;
$R^5$ is selected from at least one group consisting of hydrogen, and -NHR; wherein R is selected from at least one group consisting of substituted or unsubstituted C1-C20 alkyl, C3-C6 cycloalkyl, C3-C12 azacyclic alkyl, and C3-C12 azacyclic alkyl substituted with C1-C6 alkyl; wherein substituent of substituted C1-C20 alkyl is selected from at least one group consisting of C3-C6 cycloalkyl, amino, amino substituted with C1-C12 alkyl, C3-C12 cycloamine group, C3-C12 azacyclic alkyl, and C3-C12 azacyclic alkyl substituted with C1-C6 alkyl;
$X^-$ is selected from salt-forming anion.

2. The cryptolepine salt derivative according to claim 1, wherein $R^1$ is selected from at least one group consisting of hydrogen, C1-C3 alkyl, C1-C3 alkoxy, halogen, and cyano;

$R^2$ is selected from at least one group consisting of hydrogen, p-toluenesulfonyl, benzenesulfonyl, benzyloxycarbonyl, tert-butoxycarbonyl and benzyl;
$R^3$ is selected from at least one group consisting of substituted or unsubstituted C1-C6 alkyl, and C6-C12 aromatic group; wherein substituent of substituted C1-C6 alkyl is selected from at least one group consisting of C3-C6 cycloalkyl;
$R^4$ is selected from at least one group consisting of hydrogen, C1-C3 alkyl, C1-C3 alkoxy, halogen, C1-C3 perfluoroalkyl, and C2-C5 ester group;
$R^5$ is selected from at least one group consisting of hydrogen and -NHR; wherein R is selected from at least one group consisting of substituted or unsubstituted C1-C12 alkyl, C3-C6 cycloalkyl, C3-C6 azacyclic alkyl, and C3-C6 azacyclic alkyl substituted with C1-C3 alkyl; wherein substituent of substituted C1-C12 alkyl is selected from at least one group consisting of C3-C6 cycloalkyl, amino, amino substituted with C1-C6 alkyl, C3-C6 cycloamine group, C3-C6 azacyclic alkyl, and C3-C6 azacyclic alkyl substituted with C1-C3 alkyl;
$X^-$ is selected from at least one group consisting of $Cl^-$, $Br^-$, $I^-$, $CH_3COO^-$, $NO_3^-$, $HSO_4^-$, $H_2PO_4^-$, $BF_4^-$, and $SbF_6^-$.

3. The cryptolepine salt derivative according to claim 2, wherein,

situation 1: when $X^-$ is $Cl^-$, it is satisfied that:
$R^1$ is selected from at least one group consisting of hydrogen, methyl, methoxy, F, Cl, Br, I, and cyano; $R^2$ is selected from at least one group consisting of hydrogen, p-toluenesulfonyl, and benzenesulfonyl; $R^3$ is selected from at least one group consisting of methyl, 2,2-dimethylpropyl, cyclopropylmethyl, cyclohexylmethyl, and benzyl; $R^4$ is selected from at least one group consisting of hydrogen, methyl, methoxy, F, Cl, Br, I, trifluoromethyl,

and methyl formate group; $R^5$ is hydrogen;
situation 2: when $X^-$ is $I^-$, it is satisfied that:

$R^1$, $R^2$, $R^4$ is hydrogen, $R^3$ is methyl, $R^5$ is selected from at least one of -NHR, wherein R is selected from at least one group consisting of substituted or unsubstituted C1-C12 alkyl, C3-C6 cycloalkyl, C3-C6 azacyclic alkyl, and C3-C6 azacyclic alkyl substituted with C1-C3 alkyl; wherein substituent of substituted C1-C12 alkyl is selected from at least one group consisting of C3-C6 cycloalkyl, amino, amino substituted with C1-C6 alkyl, C3-C6 cycloamine group, C3-C6 azacyclic alkyl, and C3-C6 azacyclic alkyl substituted with C1-C3 alkyl;
Preferably, referring to the situation 2, when $X^-$ is $I^-$, it is satisfied that: $R^1$, $R^2$, $R^4$ is hydrogen, $R^3$ is methyl, $R^5$ is selected from -NHR, wherein R comprises at least one group consisting of linear or branched C3-C12 alkyl, C3-C6 cycloalkyl, methyl substituted with C3-C6 cycloalkyl, propyl substituted with C3-C6 cycloalkyl, substituted or unsubstituted C2-C6 alkylamino, tetrahydropyrrole group, piperidyl, piperidyl methyl, n-methylpiperidyl, and n-methyl piperidine methyl; wherein substituent of substituted C2-C6 alkylamino is selected from at least one group consisting of methyl, ethyl, and

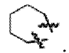.

**4.** The cryptolepine salt derivative according to any one of claims 1-3, wherein R is selected from at least one group consisting of n-propyl, n-amyl, isoamyl, n-dodecyl, 2-ethylhexyl, cyclopropyl, cyclopentyl, cyclohexyl, cyclopropylmethyl, cycloamylmethyl, cyclohexylmethyl, cyclopropylethyl, cycloamylethyl, cyclohexylethyl, cyclopropylpropyl, cycloamylpropyl, cyclohexyl propyl, aminomethyl, aminoethyl, aminopropyl, aminobutyl, 2-aminopropyl, 2-methyl-2-amino-propyl, $-CH_2CH_2-NH-CH_2CH_3$, $-CH_2CH_2CH_2-NH-CH_3$, $-CH_2C(CH_3)_2-CH_2-N(CH_3)_2$,

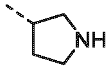, 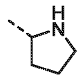,

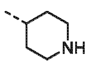, 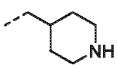, 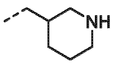, 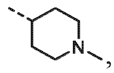, 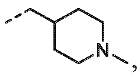, 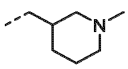, 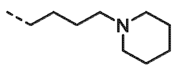, and 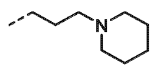.

**5.** The cryptolepine salt derivative according to any one of claims 1-4, wherein the cryptolepine salt derivative comprises at least one of the compounds shown in formula (I-1) to formula (I-71):

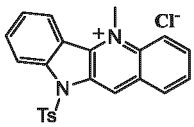
I-1

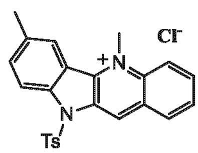
I-2

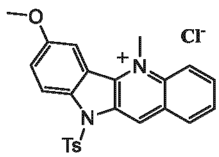
I-3

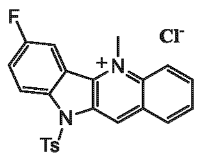
I-4

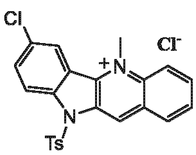
I-5

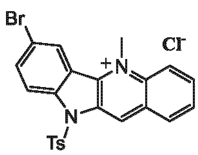
I-6

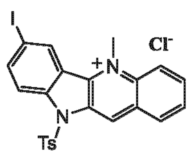
I-7

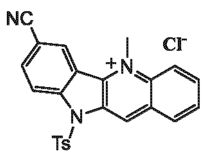
I-8

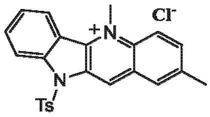
I-9

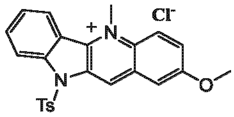
I-10

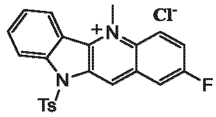
I-11

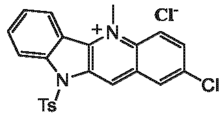
I-12

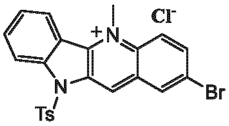
I-13

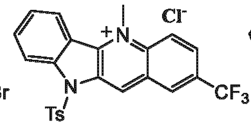
I-14

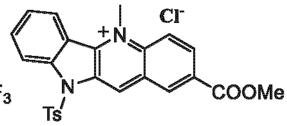
I-15

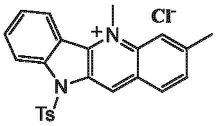
I-16

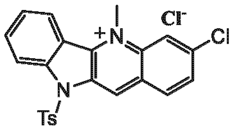
I-17

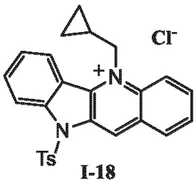
I-18

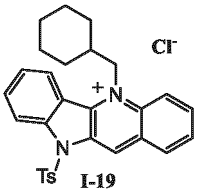
I-19

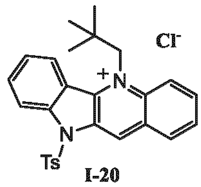
I-20

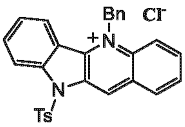
I-21

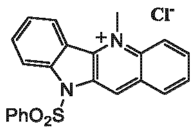
I-22

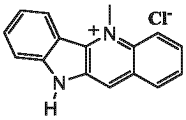
I-23

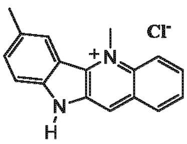
I-24

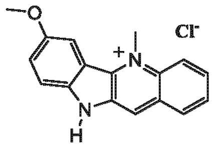
I-25

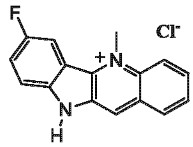
I-26

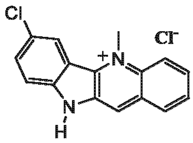
I-27

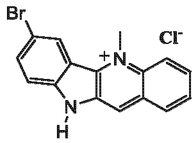
I-28

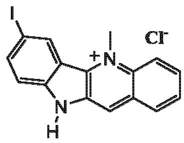
I-29

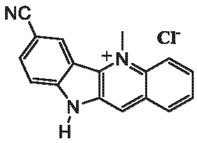
I-30

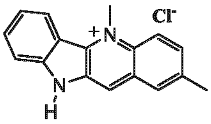
I-31

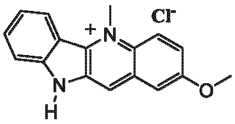
I-32

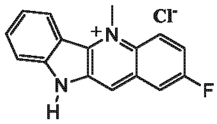
I-33

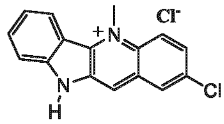
I-34

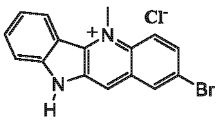
I-35

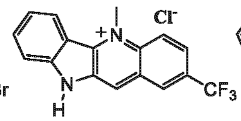
I-36

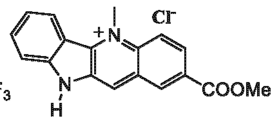
I-37

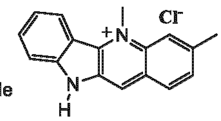
I-38

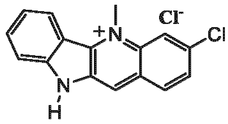
I-39

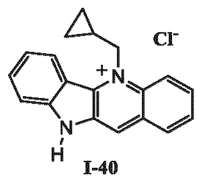
I-40

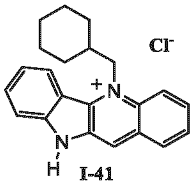
I-41

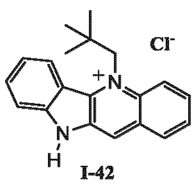
I-42

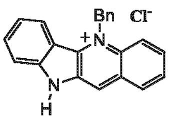
I-43

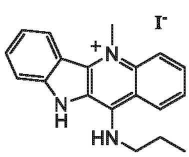
I-44

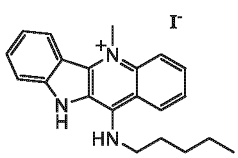
I-45

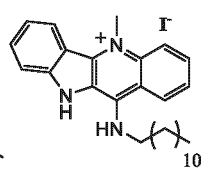
I-46

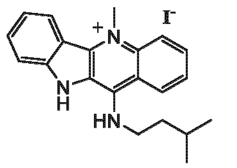
I-47

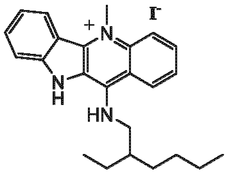
I-48

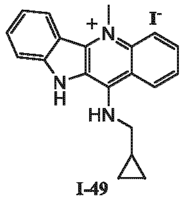
I-49

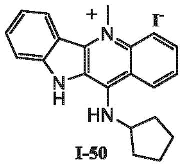
I-50

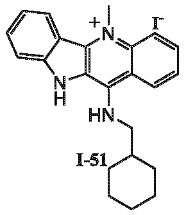
I-51

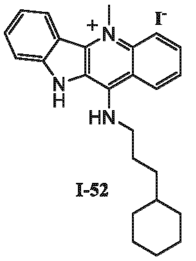
I-52

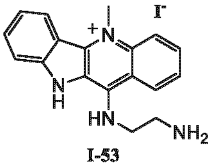
I-53

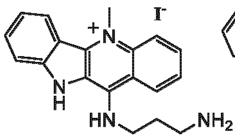
I-54

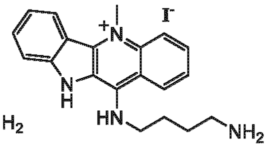
I-55

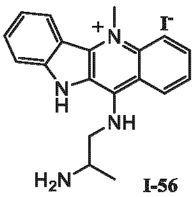
I-56

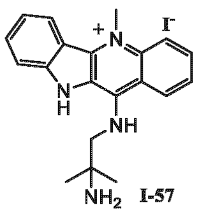
I-57

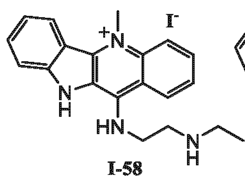
I-58

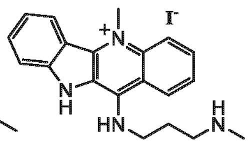
I-59

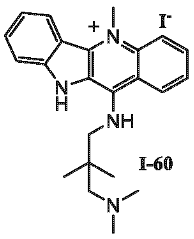
I-60

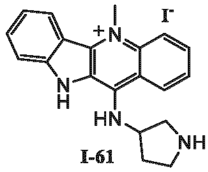
I-61

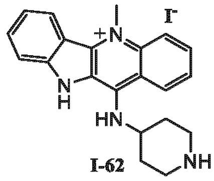
I-62

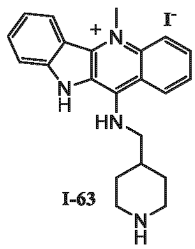
I-63

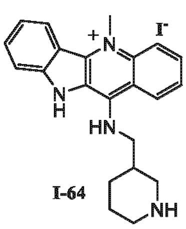
I-64

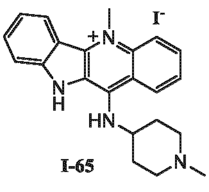
I-65

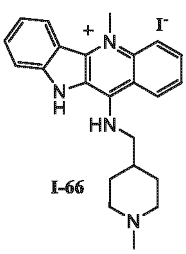
I-66

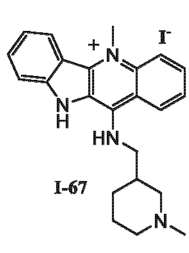
I-67

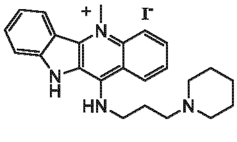
I-68

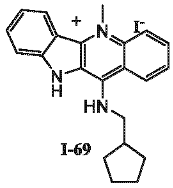
I-69

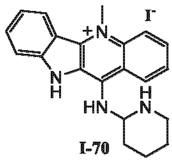
I-70

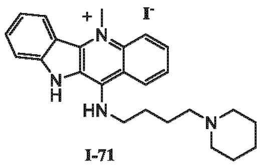
I-71

.

6. The cryptolepine salt derivative according to claim 5, wherein the cryptolepine salt derivative comprises at least one of the following compounds:

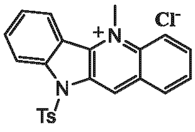
I-1

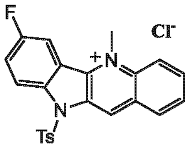
I-4

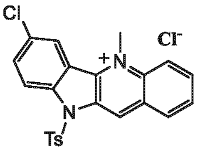
I-5

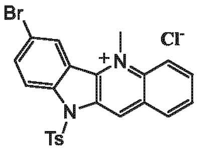
I-6

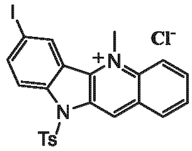
I-7

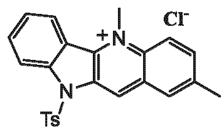
I-9

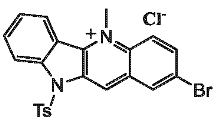
I-13

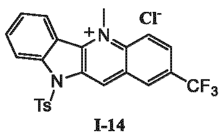
I-14

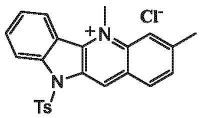
I-16

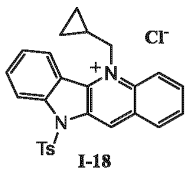
I-18

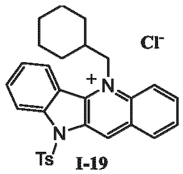
I-19

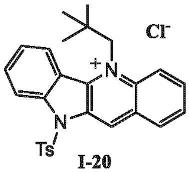
I-20

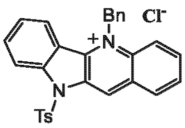
I-21

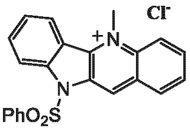
I-22

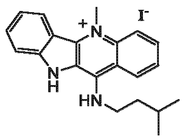
I-47

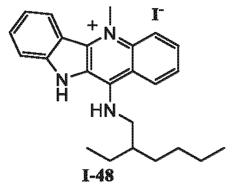
I-48

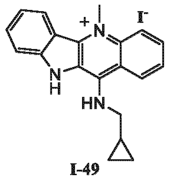
I-49

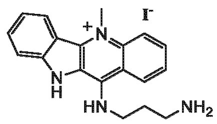
I-54

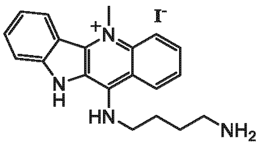
I-55

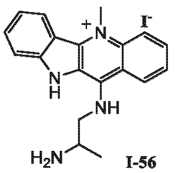
I-56

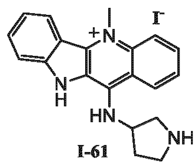
I-61

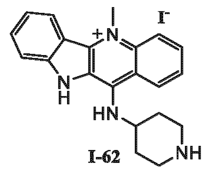
I-62

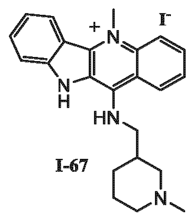
I-67

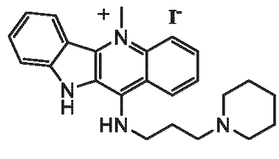
I-68

.

7. A preparation method of the cryptolepine salt derivative according to any one of claims 1-6, comprising:

method (1): when $R^5$ is hydrogen, in presence of catalyst and formylation reagent, making compound A

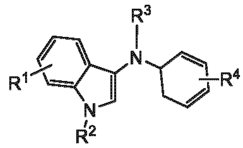

undergo electrophilic substitution reaction, thus to obtain the cryptolepine salt derivative;
method (2): when $R^5$ is selected from -NHR, making compound B

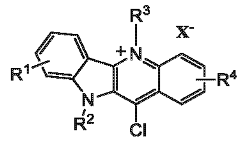

undergo nucleophilic substitution reaction with $RNH_2$ to obtain the cryptolepine salt derivative.

**8.** The preparation method according to claim 7, comprising:

method (1) : when $R^5$ is hydrogen, in presence of catalyst and formylation reagent, making compound A

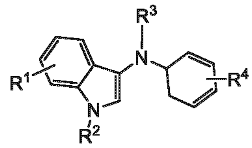

undergo electrophilic substitution reaction with imide salt, thus to obtain the cryptolepine salt derivative;
method (2) : when $R^5$ is selected from -NHR, making compound B

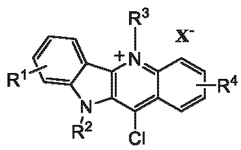

undergo nucleophilic substitution reaction with $RNH_2$ under a condition of heating reflux by using ethyl acetate as solvent to obtain the cryptolepine salt derivative.

**9.** The preparation method according to claim 7 or 8, in the method (1), the catalyst is selected from at least one of $POCl_3$, $SOCl_2$, $ZnCl_2$ and $COCl_2$, preferably selected from at least one of $POCl_3$ and $SOCl_2$;

preferably, the formylation reagent is selected from at least one of N-substituted formamides, preferably selected from at least one of N,N-Dimethylformamide, N-Methyl-N-phenylformamide, N,N-Dipropylformamide, and N-ethyl-N-methylformamide, more preferably selected from at least one of N,N-Dimethylformamide and N-Methyl-N-phenylformamide;
preferably, molar ratio of the compound A and the catalyst is 1: 1.2-3, preferably is 1: 1.5-2.5;
preferably, the compound A is provided in solution when added to reaction system, wherein concentration of the compound A is 0.2-1mmol/mL, preferably is 0.4-0.6mmol/mL;
preferably, temperature of the electrophilic substitution reaction is -20°C to 30°C, preferably is -16°C to 25°C; time of the electrophilic substitution reaction is 0.5-4h, preferably is 1-2h;
preferably, the imide salt is an intermediate

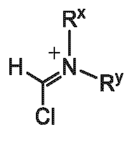

obtained by a reaction of the catalyst and the formylation reagent, wherein $R^x$ and $R^y$ are two substituents on the N position of N-substituted formamide, respectively.

**10.** The preparation method according to claim 7 or 8, in the method (2), temperature of the heating reflux is 90-150°C, preferably is 110-130°C, time of the heating reflux is 10-24h, preferably is 15-17h;
Preferably, molar ratio of the compound B to $RNH_2$ is 1:1-5, preferably is 1:1-3.

**11.** Use of the cryptolepine salt derivative according to any one of claims 1-6 in prevention and control of plant viruse.

**12.** Use of the cryptolepine salt derivative according to any one of claims 1-6 in sterilization.

**INTERNATIONAL SEARCH REPORT**

International application No. **PCT/CN2024/104657**

**A. CLASSIFICATION OF SUBJECT MATTER**

C07D471/04(2006.01)i; A01N43/90(2006.01)i; A01P1/00(2006.01)i; A01P3/00(2006.01)i

According to International Patent Classification (IPC) or to both national classification and IPC

**B. FIELDS SEARCHED**

Minimum documentation searched (classification system followed by classification symbols)

IPC: C07D, A01N, A01P

Documentation searched other than minimum documentation to the extent that such documents are included in the fields searched

Electronic data base consulted during the international search (name of data base and, where practicable, search terms used)

CNTXT, ENTXTC, WPABS, WPABSC, STN (REGISTRY, CAPLUS, MARPAT): 南开大学, 汪清民, 宋红健, 周盼, 张静静, 刘玉秀, 白叶藤碱盐, 衍生物, 抗菌, 抑菌, 杀菌, 抗病毒, NANKAI UNIVERSITY, chrysocryptolepine salt, derivative?, viru+, steriliz+, antibacterial, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| PX | CN 117886815 A (NANKAI UNIVERSITY) 16 April 2024 (2024-04-16)<br>claims 1-10, and description, paragraph 0044 | 1-12 |
| PX | CN 117683031 A (NANKAI UNIVERSITY) 12 March 2024 (2024-03-12)<br>claims 1-10, and description, paragraphs 0040-0043 | 1-12 |
| PX | ZHOU, Pan et al. "Intramolecular Trapping of an Iminium Salt: Rapid Construction of Quindoline Derivatives"<br>*Chemical Communications,* Vol. 60, No. (3), 04 December 2023 (2023-12-04), ISSN: 1359-7345,<br>pp. 292-295 | 1-12 |
| X | Registry. "RN 1622068-84-1 etc."<br>*STN,* 05 September 2014 (2014-09-05),<br>pp. 1-31 | 1-5 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **23 October 2024** | **30 October 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/104657**

**C. DOCUMENTS CONSIDERED TO BE RELEVANT**

| Category* | Citation of document, with indication, where appropriate, of the relevant passages | Relevant to claim No. |
|---|---|---|
| A | SUN, Ning et al. "Antibacterial Activity of N-methylbenzofuro[3,2-b]quinoline and N-methylbenzoindolo[3,2-b]-quinoline Derivatives and Study of Their Mode of Action" *European Journal of Medicinal Chemistry,* Vol. vol. 135, 15 April 2017 (2017-04-15), ISSN: 0223-5234, pp. 1-11 | 1-12 |
| A | CN 104447772 A (THE FIRST AFFILIATED HOSPITAL OF ANHUI MEDICAL UNIVERSITY) 25 March 2015 (2015-03-25) claims 1-8 | 1-12 |
| A | CN 112106779 A (LANZHOU UNIVERSITY) 22 December 2020 (2020-12-22) claims 1-6 | 1-12 |
| A | CN 112438271 A (LANZHOU UNIVERSITY) 05 March 2021 (2021-03-05) claims 1-6 | 1-12 |

Form PCT/ISA/210 (second sheet) (July 2022)

**INTERNATIONAL SEARCH REPORT**

International application No.
**PCT/CN2024/104657**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **12**
because they relate to subject matter not required to be searched by this Authority, namely:

Claim 12 relates to the use of cryptolepine salt derivative in sterilization. The present search report is provided on the basis of the use of the cryptolepine salt derivative in sterilization in the field of agricultural protection.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/104657**

| Patent document cited in search report | Publication date (day/month/year) | Patent family member(s) | Publication date (day/month/year) |
|---|---|---|---|
| CN 117886815 A | 16 April 2024 | None | |
| CN 117683031 A | 12 March 2024 | None | |
| CN 104447772 A | 25 March 2015 | None | |
| CN 112106779 A | 22 December 2020 | None | |
| CN 112438271 A | 05 March 2021 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311530160
- CN 202311530564


### Non-patent literature cited in the description


- *Tetrahedron Lett.*, 1998, vol. 39, 6465-6466
- *J. Med. Chem.*, 1998, vol. 41 (15), 2754-2764
- *J. Ethnopharmacol.*, 2005, vol. 100 (1-2), 67-71